# EUROPEAN PATENT SPECIFICATION

(11) **EP 1 017 805 B1**
(45) Date of publication and mention of the grant of the patent: **09.02.2005**
(21) Application number: 98911476.4
(22) Date of filing: 04.03.1998
(51) Int. Cl.: C12N 15/12, C07K 14/47, G01N 33/574, C12Q 1/68, A61K 48/00, C12N 15/86, C07K 16/18, A01K 67/027

(54) **MEN1, THE GENE ASSOCIATED WITH MULTIPLE ENDOCRINE NEOPLASIA TYPE 1, MENIN POLYPEPTIDES, AND USES THEREOF**
MEN1, MIT TYP 1 MULTIPLER ENDOKRINER NEOPLASIE ASSOZIIERTES GEN, MENINPOLYPEPTIDE UND DEREN VERWENDUNGEN
GENE MEN1 ASSOCIE A UNE ADENOMATOSE POLYENDOCRINIENNE DE TYPE 1, POLYPEPTIDES DE MENINE ET UTILISATION DE CEUX-CI

(30) Priority: 05.03.1997 US 40269 P
(43) Date of publication of application: 12.07.2000
(73) Proprietor: THE GOVERNMENT OF THE UNITED STATES OF AMERICA as represented by the SECRETARY OF THE DEPARTMENT OF HEALTH AND HUMAN SERVICES, Bethesda, MD 20892-9902 (US)
(72) Inventor: CHANDRASEKHARAPPA, Settara C., Lab.of Gene Transf., Bethesda, MD 20892 (US); GURU, Siradanahalli, C., Lab. of Gene Transf., Bethesda, MD 20892 (US); MANICKAM, Pachiappan, Lab.of Gene Transf., Bethesda, MD 20892 (US); COLLINS, Francis, S., Lab. Gene Transf., Bethesda, MD 20892 (US); EMMERT-BUCK, Michael, R., Lab. of Pathology, Bethesda, MD 20892 (US); DEBELENKO, Larisa, V., Lab. of Pathology, Bethesda, MD 20892 (US); LUBENSKY, Irina, A., Lab. of Pathology, Bethesda, MD 20892 (US); LIOTTA, Lance, A., Lab. of Pathology, Bethesda, MD 20892 (US); AGARWAL, Sunita, K., Metabolic Diseases Branch, Bethesda, MD 20892 (US); SPIEGEL, Allen, M., Metabolic Diseases Branch, Bethesda MD 20892 (US); BURNS, A., Lee, Metabolic Diseases Branch, Bethesda MD 20892 (US); MARX, Stephen, J., Metabolic Diseases Branch, Bethesda MD 20892 (US)
(74) Representative: Dzieglewska, Hanna Eva
(86) International application number: PCT/US1998/004258
(87) International publication number: WO 1998/039439

(56) References cited:
- LARSSON C ET AL: "Multiple endocrine neoplasia type 1 gene maps to chromosome11 and is lost in insulinoma" NATURE, vol. 332, 3 March 1988, pages 85-87, XP002069543
- CHANDRASEKHARAPPA S ET AL: "Positional cloning of the gene for multiple endocrine neoplasia-type 1" SCIENCE., vol. 276, 18 April 1997, pages 404-407, XP002069544
- KEDRA D ET AL: "The germinal center kinase gene and a novel CDC25-like gene are located in the vicinity of the PYGM gene on 11q13" HUM. GENET., vol. 100, October 1997, pages 611-619, XP002069545
- DATABASE EMEST13 E.M.B.L. DATABASE Accession Number: AA168218, 21 December 1996 MARRA M ET AL: "Mus musculus cDNA clone 608138" XP002070015
- DATABASE EMEST13 E.M.B.L. DATABASE Accession Number: W89897, 7 July 1996 MARRA M ET AL: "Mus musculus cDNA clone 420339" XP002070016
- DATABASE EMEST12 E.M.B.L. DATABASE Accession Number: AA000099, 20 July 1996 MARRA M ET AL: "Mus musculus cDNA clone 425424" XP002070017

## Description

### FIELD OF THE INVENTION

This invention relates to the discovery of a novel tumor suppressor gene which is associated with multiple endocrine neoplasia type 1. The gene has been designated *MEN1* and the gene product is menin. The lack of a functional menin polypeptide, either by absence of the protein, its alteration and/or associated mutations in the corresponding gene, have been identified in individuals with familial multiple endocrine neoplasia type I (FMEN1) and suffering from multiple endocrine neoplasia type 1. The identification of *MEN1* as a marker for multiple endocrine neoplasia type 1 has diagnostic uses in cancer and in endocrine disease, as well as application for gene therapy.

### BACKGROUND OF THE INVENTION

Familial multiple endocrine neoplasia type 1 (FMEN1) is an autosomal dominant familial cancer syndrome characterized by the frequent occurrence of tumors in the parathyroids, gastro-intestinal endocrine tissues (*e*.*g*., gastrinomas, tumors of the endocrine duodenum), enteropancreatic endocrine tissues, the anterior pituitary, and occasionally other sites. In keeping with the hypothesis originally articulated by Knudson for retinoblastoma (see Knudson (1971) *Proc. Natl. Acad Sci. U*.*S*.*A*. 68:820), most of the genes responsible for familial cancer syndromes are of the tumor suppressor type. In such a circumstance, affected individuals have inherited one altered copy of the responsible gene from an affected parent (they are heterozygotes), but the tumors have lost the remaining functional copy (the "wild-type allele") as a somatic event (becoming non-functional homozygotes). Germ-line homozygocity would be fatal. Thus the inheritance pattern of the syndrome is dominant, but the mechanism of tumorigenesis is recessive.

Finding the gene responsible for such a cancer syndrome would provide a new window into the mechanism of tumorigenesis, would facilitate accurate early diagnosis, and would, through screening, identify individuals predisposed to cancer. In the case of MEN1, a patient in an affected kindred manifesting any one of the classic features of the syndrome is at risk for the development of the other associated tumors. According to standard medical practice, periodic screening of both affected individuals and unaffected relatives is considered essential. Standard practice recommends that this be done at least biannually and include, e.g., review of the history for symptoms suggestive of peptic ulcer disease, diarrhea, nephrolithiasis, hypoglycemia, and hypopituitarism; physical examination for features of acromegaly and subcutaneous lipomas; and measurement of serum Ca, phosphate (PO4), gastrin, and prolactin. Further laboratory testing, CT or MRI of the sella turcica, and other diagnostic maneuvers should also be performed when clinically indicated. Prior to this invention, no protein or gene-based diagnostic test was available.

The significance of discovering such a tumor suppressor gene extends beyond affected pedigrees, as the same tumor suppressor gene is often found to play a role (by somatic mutation of both alleles in individuals born homozygous for a wild-type, functional allele) in sporadic cases of various neoplasms. These non-hereditary tumors are typically endocrine tumors. Identification of non-hereditary mutations can give information about the origin of the tumor and its prognosis. Furthermore, treatments arising from the gene's discovery will lead to the amelioration and/or prevention of such tumors.

Thus, there exists a great need to identify the gene associated with MEN1 and FMEN1; as characterization such a gene which would allow identification of those individuals with MEN1, provide lifesaving diagnostic tests, therapeutic treatment regimens, and prognostic evaluations of individuals with FMEN1 and individuals with somatic mutations involving both alleles resulting in non-hereditary tumors. The present invention fulfills these and other needs.

### BRIEF DESCRIPTION OF THE FIGURES

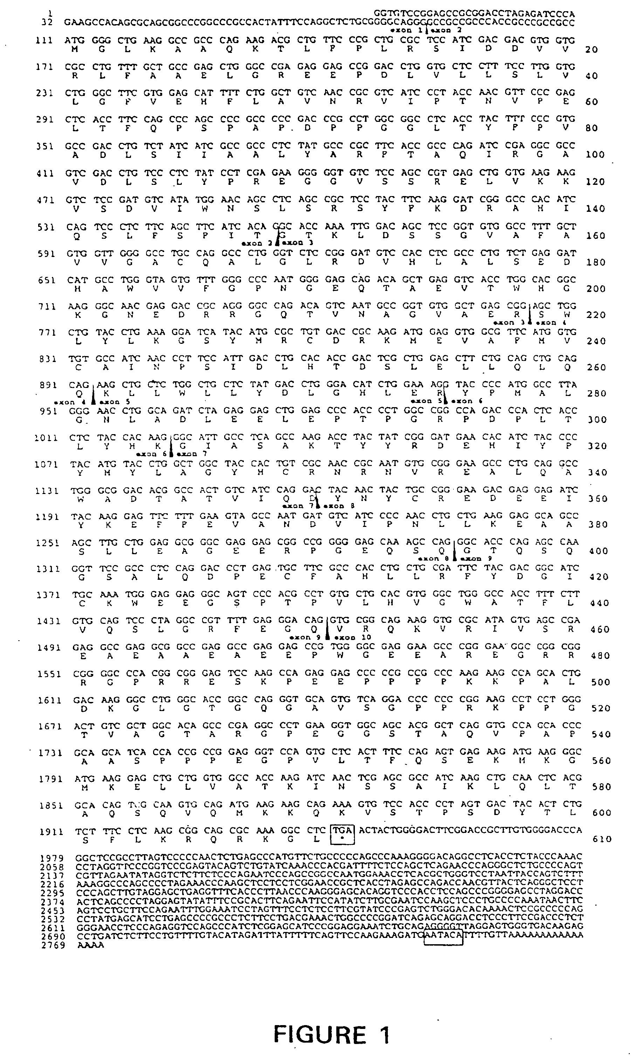

Figure 1 shows the nucleotide sequence and of an apparently full length *MEN1* cDNA as derived from a leukocyte cDNA clone (SEQ ID NO:1); and the corresponding predicted amino acid sequence (SEQ ID NO:2). The boundaries of the exons are shown, as identified by comparison with the complete genomic sequence of a 9.2 kb segment containing all 10 exons (SEQ ID NO:3). The first exon is completely untranslated. The ATG at nucleotide 111 is predicted to be the initiation codon, since it is associated with an excellent Kozak consensus sequence, and there are no other in-frame ATG codons upstream of this (an in-frame stop occurs at nucleotide 20). The stop codon (TGA) and the proposed polyadenylation signal sequence (AATACA) are boxed. The polyadenylation signal is a variant from the usual AATAAA, and appears located closer than usual to the string of adenosines; however, the first eight of these are also represented in genomic DNA.

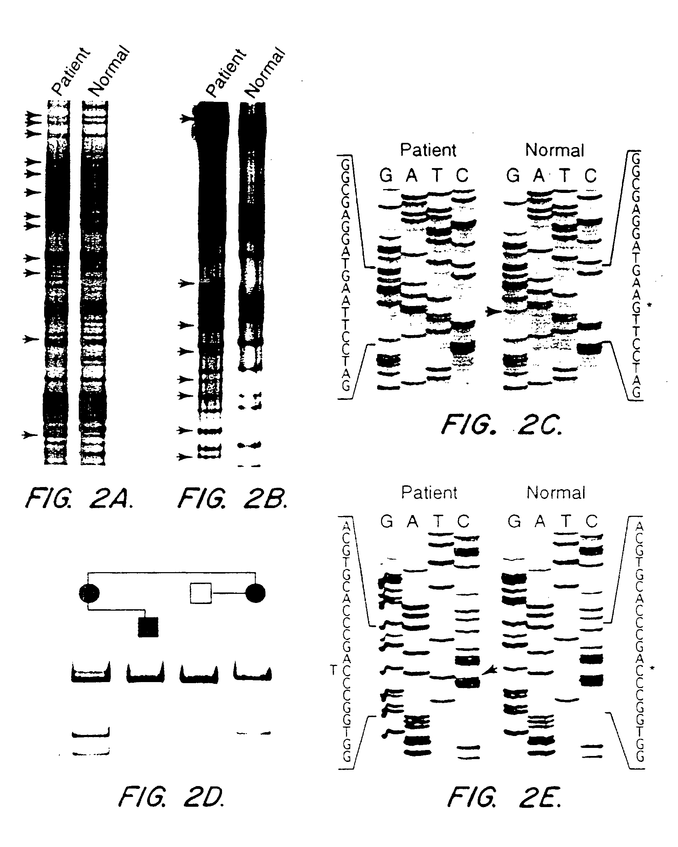

Figure 2 shows the detection of frameshift and nonsense mutations. **(A)** Analysis of exon 2 in a MEN 1 patient and a normal control, using dideoxy fingerprinting (ddF) to reveal pattern differences (arrows) indicative of a possible mutation. **(B)** Abnormal ddF pattern in exon 9 from a different patient. (C) Identification of a single nucleotide deletion by sequencing of a cloned exon 2 PCR product from the patient whose ddF pattern is shown in (A). The sequence shown is of the antisense strand; the mutation is 512delC. This frameshift mutation was confirmed by detecting the presence of a new *AflII* site in PCR-amplified exon 2 from this patient and two affected relatives **(D)**. **(E)** Direct sequencing of the exon 9 PCR product from panel (B), revealing the presence of a heterozygous C to T (C => T) substitution. Again the sequence is of the antisense strand; the mutation creates a stop codon: TGG to TAG or W436X (TGG => TAG or W436X).

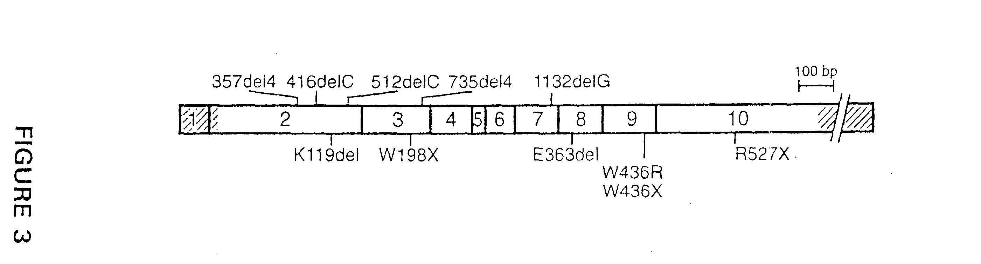

Figure 3 show a summary of mutations identified in 15 unrelated MEN1 patients. The locations of the five frameshift mutations are shown above a diagram of the *MEN1* gene, with the exons numbered; crosshatched areas are untranslated. Two in-frame deletions of a single amino acid, three nonsense mutations, and one missense mutation (W436R) are shown below the gene diagram. The 416delC and 512delC mutations were each encountered twice. Mutation abbreviations follow standard nomenclature, e.g., as described by Beaudet (1993) "A suggested nomenclature for designating mutations," Hum *Mutat*. 2(4):245-248.

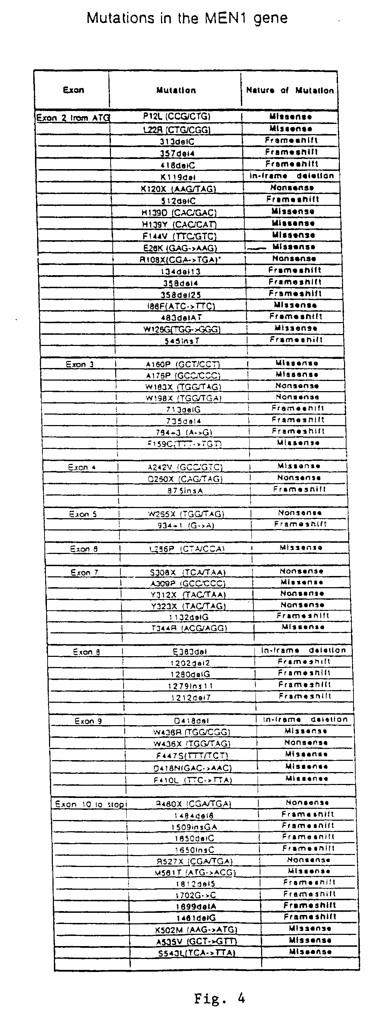

Figure 4 shows a summary of mutations identified in unrelated MEN1 patients. Abbreviations follow standard nomenclature (Beaudet (1993) *supra*).

### SUMMARY OF THE INVENTION

This invention provides for an isolated or recombinant nucleic acid associated with the presence of multiple endocrine neoplasia type 1, wherein said nucleic acid encodes a protein defined as having a calculated molecular weight of about 67.5 kDa; and (a) specifically binding to an antibody raised against an protein with a sequence as set forth in SEQ ID NO:2; or (b) having at least 60% amino acid sequence identity to a protein with a sequence as set forth in SEQ ID NO:2. In various embodiments, the isolated or recombinant nucleic acid can further comprises non-coding sequence; and the non-coding sequence can comprises introns. In one embodiment, the isolated or recombinant nucleic acid has the sequence as set forth in SEQ ID NO:3.

The invention also provides for an isolated or recombinant nucleic acid which encodes a protein having at least 80% amino acid sequence identity to a protein with a sequence as set forth in SEQ ID NO:2. In alternative embodiments, the isolated or recombinant nucleic acid encodes a menin protein with a sequence as set forth in SEQ ID NO:2; or, the isolated or recombinant nucleic acid has the sequence as set forth in SEQ ID NO:1.

This invention further provides for an isolated or recombinant protein defined as having a calculated molecular weight of about 67.5 kDa; and (a) specifically binding to an antibody raised against an protein with a sequence as set forth in SEQ ID NO:2; or (b) having at least 60% amino acid sequence identity to a protein with a sequence as set forth in SEQ ID NO:2. In various embodiments, the isolated or recombinant protein has at least 80% amino acid sequence identity to a protein with a sequence as set forth in SEQ ID NO:2; or, an isolated or recombinant protein having a sequence as set forth in SEQ ID NO:2.

The invention further provides for an antibody, specifically immunoreactive under immunologically reactive conditions, to a protein comprising SEQ ID NO:2; and, an antibody, specifically immunoreactive under immunologically reactive conditions, to a protein, wherein the protein is encoded by a nucleic acid encoding a protein defined as having a calculated molecular weight of about 67.5 kDa; and (a) specifically binding to an antibody raised against an protein with a sequence as set forth in SEQ ID NO:2; or (b) having at least 60% amino acid sequence identity to a protein with a sequence as set forth in SEQ ID NO:2.

This invention further provides for methods of detecting the presence of menin in human cells and tissues, said method comprising: (i) isolating a biological sample from a human being tested for menin; (ii) contacting the biological sample with a menin specific reagent; and, (iii) detecting the level of menin specific reagent that selectively associates with the sample. The method is contemplated for a variety of different purposes including sporadic (non-hereditary) cancers arising in any cell or tissue from mutations of *MEN1* or loss of heterozygosity of wild type *MEN1*, as in multiple endocrine neoplasia type 1 (MEN1). The methods include nucleic acid hybridization technology, amplification of nucleic acid technology and immunoassays.

In various embodiments, in the method of detecting the presence of menin in human cells and tissues, the menin specific reagent is selected from the group comprising: menin specific antibodies, *MEN1* amplification primers and nucleic acid probes which selectively bind to *MEN1*. In this method, the contacting step can also use a menin specific antibody. The human from which the sample is isolated is suspected of being at risk from multiple endocrine neoplasia type 1 (MEN1). In this method, the contacting step can use a *MEN1* specific PCR primer pair that amplifies a region of the *MEN1* gene in which a mutation has been associated with multiple endocrine neoplasia type 1 (MEN1).

The invention also provides for a method for detecting in a test sample the presence or absence of a mutation in a nucleotide sequence essentially encoding human menin comprising: contacting said test sample suspected of lacking a *MEN1* allele or containing a gene encoding a mutant form of the human menin with a first oligonucleotide having a sequence competent to discriminate between the wild type gene and the missing *MEN1* allele or mutant form; and, b) detecting the formation of a duplex between the gene and the first oligonucleotide sequence. Such assays would include those wherein the first oligonucleotide is unable to bind to the wild-type *MEN1* gene under hybridization conditions in which the first nucleotide binds to the mutant sequence of *MEN1*.

This invention also provides for kits for detecting in a test sample the presence or absence of a mutation in a nucleotide sequence corresponding to the wild type allele encoding menin comprising: a) a container holding a first oligonucleotide sequence whereby said first nucleotide sequence is capable of discriminating between the wild type gene and the mutant form; and b) a container holding a reagent for detecting the formation of a duplex between the gene and the first nucleotide sequence. The kit can further comprising amplification primer pairs specifically binding to a human genomic DNA sequence containing *MEN1*.

The invention further provides for a kit for detecting the presence or absence of menin in persons at risk for multiple endocrine neoplasia type 1 comprising a first container containing at least one antibody specific for a wild type menin or a mutant menin, and a second container containing an antigen that specifically binds to the menin specific antibody in the first container. The antibody can can detect the presence of a wild-type menin protein or the presence of a mutated menin protein. Kits for detecting any menin polypeptide aberration using immunoassays are also included in this invention for all of the various purposes recited above.

The invention also provides for a transfected cell comprising a heterologous nucleic acid encoding a menin protein or subsequence thereof. In one embodiment, the invention provides for a transfected cell into which an exogenous nucleic acid sequence has been introduced, the exogenous nucleic acid specifically hybridizing under stringent conditions to a nucleic acid with: a nucleic acid sequence as set forth in SEQ ID NO:1 or SEQ ID NO:3; or, a nucleic acid encoding a protein defined as having a calculated molecular weight of about 67.5 kDa; and (a) specifically binding to an antibody raised against an protein with a sequence as set forth in SEQ ID NO:2; or (b) having at least 60% amino acid sequence identity to a protein with a sequence as set forth in SEQ ID NO:2; and, the cell expresses the exogenous nucleic acid as a menin protein. The transfected cell of the invention can comprise a nucleic acid as set forth in SEQ ID NO:1 or SEQ ID NO:3. The transfected cell can be a human cell.

The invention provides for an organism into which an exogenous nucleic acid sequence has been introduced, the exogenous nucleic acid specifically hybridizing under stringent conditions to a nucleic acid with: a sequence as set forth in SEQ ID NO:1; or, a nucleic acid encoding a protein defined as having a calculated molecular weight of about 67.5 kDa; and (a) specifically binding to an antibody raised against an protein with a sequence as set forth in SEQ ID NO:2; or (b) having at least 60% amino acid sequence identity to a protein with a sequence as set forth in SEQ ID NO:2; and, the organism expresses the exogenous nucleic acid as a menin protein. In one embodiment, the organism comprises an exogenous nucleic acid as set forth in SEQ ID NO:1 or SEQ ID NO:3.

The invention also provides for an expression cassette comprising a nucleic acid encoding a menin polypeptide, wherein the nucleic acid is operably linked to a promoter and comprises a nucleic acid encoding a menin polypeptide. In one embodiment, the expression cassette further comprises an expression vector.

Finally, this invention also includes research uses for the MEN 1 gene and its protein product, menin. Thus the detection of either the gene or gene product for detection of cells producing or suspected of producing menin are also contemplated for any use including simply identifying menin producing cells from non-menin producing cells such as animal or human cells from bacterial cells.

The invention also embraces the use of antisense methods for studying menin in animals and cells. Typically any time a gene is identified, it can be studied by knocking out the gene in an animal and observing the effect on the animal phenotype. Knockouts can be achieved by transposons which insert by homologous recombinations, antisense or ribozymes specifically directed at disturbing the embryonic stem cells of an organism such as a mouse. Ribozymes can include any of the various types of ribozymes modified to cleave the mRNA encoding menin. Examples include hairpins and hammerhead ribozymes. Finally antisense moiecuies which selectively bind to the menin mRNA are expressed via expression cassettes operably linked to subsequences of the menin gene and generally comprise 20-50 base long sequences in opposite orientation to the mRNA to which they are targeted.

### DEFINITIONS

"Amplification" primers are oligonucleotides comprising either natural or analog nucleotides that can serve as the basis for the amplification of a select nucleic acid sequence. They include both polymerase chain reaction primers and ligase chain reaction oligonucleotides.

"Antibody" refers to a polypeptide substantially encoded by an immunoglobulin gene or immunoglobulin genes, or fragments thereof which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively. Antibodies exist *e.g*., as intact immunoglobulins or as a number of well characterized fragments produced by digestion with various peptidases. Thus, for example, pepsin digests an antibody below the disulfide linkages in the hinge region to produce F(ab)'_{2,} a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially an Fab with part of the hinge region (*see, Fundamental Immunology*, Third Edition, W.E. Paul, ed., Raven Press, N.Y. 1993). While various antibody fragments are defined in terms of the digestion of an intact antibody, one of skill will appreciate that such fragments may be synthesized *de novo* either chemically or by utilizing recombinant DNA methodology. Thus, the term antibody, as used herein, also includes antibody fragments either produced by the modification of whole antibodies or those synthesized *de novo* using recombinant DNA methodologies (*e.*g., single chain Fv).

"Associated" in the context of multiple endocrine neoplasia type 1 refers to relationship of the *MEN1* gene to the disease. In a classic sense, it is a suppressor gene. However it is not known if the effect of the mutation is direct or indirect upon the cell phenotype. Accordingly, we refer to the relationship as an association rather than a suppressor effect because of the lack of proof that (lack of a functional) *MEN1* is directly responsible for the cancer phenotype. This fact in no way diminishes the use of the gene in diagnosing either multiple endocrine neoplasia type 1 (MEN1, see definition, below) *per se,* or a predisposition to multiple endocrine neoplasia type 1 in an individual at risk for this disease. Individuals at risk for the disease have only one functional *MEN1* gene product (are heterozygotes for wild type *MEN1*), and have inherited the familial multiple endocrine neoplasia type 1 syndrome.

"Biological samples" refers to any tissue or liquid sample having a menin-encoding nucleic acid, including message or genomic DNA, or the *MEN1* gene product. It includes both cells with a normal complement of chromosomes and cells suspected of bearing at least one oncogenic mutation, *e.g*., in *MEN1*.

"Competent to discriminate between the wild type gene and the mutant form" means a hybridization probe or primer sequence that allows the trained artisan to detect the presence or absence of base changes, deletions or additions to a wild type nucleotide sequence encoding menin. The term "wild type gene" includes all alleles encoding for a functional gene product, such as an *MEN1* gene encoding a functional gene product, the menin polypeptide. A probe sequence is a sequence containing the site that is changed, deleted to or added to. A primer sequence will hybridize with the sequences surrounding or flanking the base changes, deletions or additions and using the gene sequence as template allow the further synthesis of nucleotide sequences that contain the base changes or additions. In addition, the probe may act as a primer. It is important to point out that this invention allows for the design of PCR primers capable to amplify entire exons. To achieve this, primers need hybridize with intron sequences. This invention provides such intron sequences. "Competent to discriminate between the wild type gene and the mutant form" also includes an immunoreactive reagent, *e.*g., an antibody, that allows the trained artisan to detect the presence or absence of a wild type (biologically active allele of) menin, as opposed to a non-functional menin.

The term "expression cassette" refers to any recombinant expression system for the purpose of expressing a nucleic acid sequence of the invention *in vitro* or *in vivo,* constitutively or inducibly, in any cell, including prokaryotic, yeast, fungal, plant, insect or mammalian cell. The term includes linear or circular expression systems. The term includes expression cassettes, *e*.*g*., vectors, that remain episomal or integrate into the host cell genome. The expression cassettes can have the ability to self-replicate or not, *i*.*e*., drive only transient expression in a cell. The term includes recombinant expression cassettes which contain only the minimum elements needed for transcription of the recombinant nucleic acid.

The phrase "genomic" refers to DNA which includes both the exon and intron regions as well as the untranslated sequences that are 5' exon 1 and 3' of exon 10 of the MEN 1 gene. Intron 1 is between exon I and exon II, intron 2 is between exon II and exon III, intron 3 is between exon III and exon IV, and intron is between exon IV and exon V and so on The term "genomic" also refers to any cis-acting transcriptional regulatory elements, *e.*g., promoters and enhancers, that regulate the expression of *MEN1*.

The term "isolated," when referring to a molecule or composition, such as, for example, a polypeptide or nucleic acid, means that the molecule or composition is separated from at least one other compound, such as a protein, other nucleic acids (*e*.*g*., RNAs), or other contaminants with which it is associated *in vivo* or in its naturally occurring state. Thus, a polypeptide or nucleic acid is considered isolated when it has been isolated from any other component with which it is naturally associated, *e.g*., cell membrane, as in a cell extract. An "isolated" composition can, however, also be substantially pure (substantially free of other cellular components). An "isolated" composition can be when the nucleic acid or protein is essentially free of other cellular components with which it is associated in the natural state. It can be in a homogeneous state, although it can also be in either a dry or aqueous solution. Purity and homogeneity are typically determined using analytical chemistry techniques such as polyacrylamide gel electrophoresis or high performance liquid chromatography. In one embodiment, the protein which is the predominant species present in a preparation is substantially purified. In particular, an isolated *MEN1* gene is separated from open reading frames which flank the gene and encode a protein other than the *MEN1* gene product. The term "purified" denotes that a nucleic acid or protein gives rise to essentially one band in an electrophoretic gel. Particularly, it means that the nucleic acid or protein is at least 85% pure, more preferably at least 95% pure, and most preferably at least 99% pure.

"*MEN1*" refers to a gene encoding the polypeptide menin. There is an expectation that the human population has a normal degree of variance in this gene (wild-type alleles encoding for functional, biologically active menin). The genomic sequence of an exemplary wild-type *MEN1* allele is set forth as SEQ ID NO:3, with the corresponding transcribed sequence (cDNA) as SEQ ID NO:1. The presence of mutations in MEN 1, producing a nonfunctional MEN1 allele (incapable of producing a biologically active menin polypeptide) is associated with the presence of MEN1, a familial cancer syndrome.

"Menin" refers to a protein encoded by an *MEN1* gene (allele). In its full length, wild-type form it is about 67.5 kilodaltons (kd), its theoretical molecular weight whose calculation is based on translation of the *MEN1* gene nucleic acid sequence. The term "menin" includes polypeptides which selectively bind to antibodies raised against *a MEN1* gene product, *e*.*g*., SEQ ID NO:2, or are encoded by nucleic acid that would selectively hybridize to the exons of SEQ ID NO:3. "Wild-type menin" includes those allelic variants which could reverse the tumorigenic phenotype.

"Menin-specific reagent" is a composition of matter that binds to either menin (*e*.*g*., an antibody) or to *MEN1* (e.g., nucleic acid probes and/or amplification primers).

"Multiple endocrine neoplasia-type 1," sometimes referred to as "MEN1" or "MEN I" or "Familial multiple endocrine neoplasia-type 1" or "FMEN1," refers a familial cancer syndrome in which affected individuals develop varying combinations of endocrine and non-endocrine tumors; including tumors of the parathyroids, pancreatic islets (insulinomas), duodenal endocrine cells, anterior pituitary, foregut carcinoids (gastrinomas), lipomas (non-endocrine), angiofibromas (non-endocrine), thyroid adenomas, adrenocortical adenomas, angiomyolipomas (non-endocrine), spinal cord ependymonas, neuroendocrine tumors of lung, thymus and stomach. Except for gastrinomas, most of the tumors are nonmetastasizing. Many have striking clinical effects because of the secretion of endocrine substances, such as gastrin, insulin, parathyroid, hormone, prolactin, growth hormone, glucagon, or adrenocorticotropic hormone. The clinical features of the MEN-I syndrome depend upon the pattern of tumor involvement in the individual patient. About 40% of reported cases have had tumors of the parathyroids, pancreas, and pituitary. Almost any combination of tumors and symptom complexes outlined above is possible.

MEN1 one type of "multiple endocrine neoplasias" (also called "Multiple Endocrine Adenomatosis, "MEA," or "Familial Endocrine Adenomatosis"), which are a group of genetically distinct familial diseases involving adenomatous hyperplasia and malignant tumor formation in several endocrine glands. Three distinct syndromes, MEN1, MEN II and MEN III, have been identified; each is inherited as an autosomal dominant trait with a high degree of penetrance, variable expressivity, and significant pleiotropism. Prior to this invention, the relationship between the genetic abnormalities and the pathogenesis of the various tumors was not understood.

The term "nucleic acid" refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g*. degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (see, *e.g.,* Batzer (1991) *Nucleic Acid Res. 19*:5081; Ohtsuka (1985) *J*. *Biol*. *Chem. 260*:2605-2608; and Cassol (1992); Rossolini (1994) *Mol*. *Cell*. *Probes* 8:91-98). The term nucleic acid is used interchangeably with gene, cDNA, and mRNA encoded by a gene.

"Nucleic acid derived from a gene" refers to a nucleic acid for whose synthesis the gene, or a subsequence thereof, has ultimately served as a template. Thus, an mRNA, a cDNA reverse transcribed from an mRNA, an RNA transcribed from that cDNA, a DNA amplified from the cDNA, an RNA transcribed from the amplified DNA, *etc.,* are all derived from the gene and detection of such derived products is indicative of the presence and/or abundance of the original gene and/or gene transcript in a sample.

"Nucleic acid probes" may be DNA or RNA fragments. DNA fragments can be prepared, for example, by digesting plasmid DNA, or by use of PCR, or synthesized by either the phosphoramidite method described by Beaucage and Carruthers, *Tetrahedron Lett.* 22:1859-1862 (1981) (Beaucage and Carruthers), or by the triester method according to Matteucci (1981) *J. Am*. *Chem. Soc.,* 103:3185 (Matteucci). A double stranded fragment may then be obtained, if desired, by annealing the chemically synthesized single strands together under appropriate conditions or by synthesizing the complementary strand using DNA polymerase with an appropriate primer sequence. Where a specific sequence for a nucleic acid probe is given, it is understood that the complementary strand is also identified and included. The complementary strand will work equally well in situations where the target is a double-stranded nucleic acid.

The phrase "a nucleic acid sequence encoding" refers to a nucleic acid which contains sequence information for a structural RNA such as rRNA, a tRNA, or the primary amino acid sequence of a specific protein or peptide. The term can also refer to a binding site for a trans-acting regulatory agent. This phrase specifically encompasses degenerate codons (*i*.*e*., different codons which encode a single amino acid) of the native sequence or sequences which may be introduced to conform with codon preference in a specific host cell.

The phrase "specifically (or selectively) binds to an antibody" or "specifically (or selectively) immunoreactive with", when referring to a protein or peptide, refers to a binding reaction which is determinative of the presence of the protein in the presence of a heterogeneous population of proteins and other biologics. Thus, under designated immunoassay conditions, the specified antibodies bind to a particular protein and do not bind in a significant amount to other proteins present in the sample. Specific binding to an antibody under such conditions may require an antibody that is selected for its specificity for a particular protein. For example, antibodies raised to a wild-type menin, such as menin with the amino acid sequence encoded in SEQ ID NO:2, can be selected to obtain antibodies (polyclonal or monoclonal) specifically immunoreactive with all wild-type menin proteins and not with non-menin proteins. Antibodies can also be raised to react only with a specific wild-type polymorphic variant of menin (encoded by an *MEN1* allele). A variety of immunoassay formats may be used to select antibodies specifically immunoreactive with a particular protein. For example, solid-phase ELISA immunoassays, Western blots, or immunohistochemistry are routinely used to select monoclonal antibodies specifically immunoreactive with a protein. See Harlow and Lane (1988) *Antibodies, A Laboratory Manual*, Cold Spring Harbor Publications, New York "Harlow and Lane"), for a description of immunoassay formats and conditions that can be used to determine specific immunoreactivity. As used herein, a specific or selective reaction will be at least twice background signal or noise; although typically it can be more than 10 to 100 times background.

The phrases "hybridizing specifically to" or "hybridizing selectively to" or "selectively or specifically hybridizes", refers to the binding, duplexing, or hybridizing of a nucleic acid molecule preferentially to a particular nucleotide sequence under stringent conditions when that sequence is present in a complex mixture (e.g., total cellular) DNA or RNA "Stringent hybridization" or "stringent conditions" and "stringent hybridization wash conditions" in the context of nucleic acid hybridization experiments, e.g., Southern and Northern hybridizations, are sequence dependent, and are different under different environmental parameters. An extensive guide to the hybridization of nucleic acids is found in Tijssen (1993) *Laboratory Techniques in Biochemistry and Molecular Biology - Hybridization with Nucleic Acid Probes* part I chapter 2 "overview of principles of hybridization and the strategy of nucleic acid probe assays", Elsevier, New York. Generally, stringent hybridization and wash conditions are selected to be about 5°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids which have more than 100 complementary residues on a filter in a Southern or Northern blot is 50% formamide with 1 mg of heparin at 42°C, with the hybridization being carried out overnight. An example of highly stringent wash conditions is: 0.15M NaCl at 72°C for about 15 minutes. An example of stringent wash conditions is a 0.2x SSC wash at 65°C for 15 minutes (*see*, Sambrook, *supra* for a description of SSC buffer). Often, a high stringency wash is preceded by a low stringency wash to remove background probe signal. An example medium stringency wash for a duplex of, *e.g*., more than 100 nucleotides, is 1x SSC at 45°C for 15 minutes. An example low stringency wash for a duplex of, *e.g*., more than 100 nucleotides, is 4 to 6x SSC at 40°C for 15 minutes. As used herein, a signal to noise ratio of 2x (or higher) than that observed for an unrelated probe in the particular hybridization assay indicates detection of a specific hybridization. Nucleic acids which do not hybridize to each other under stringent conditions can still be substantially identical if the polypeptides which they encode are substantially identical. This occurs, *e.g.,* when a copy of a nucleic acid is created using the maximum codon degeneracy permitted by the genetic code.

The terms "substantial identity" or "substantial sequence identity" as applied to nucleic acid sequences and as used herein and denote a characteristic of a polynucleotide sequence, wherein the polynucleotide comprises a sequence that has at least 85 percent sequence identity, preferably at least 90 to 95 percent sequence identity, and more preferably at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison (see detailed discussion, below). The reference sequence may be a subset of a larger sequence.

As applied to polypeptides such as menin, the terms "substantial identity" or "substantial sequence identity" mean that two peptide sequences, when optimally aligned, such as by the programs GAP or BESTFIT using default gap weights (see detailed discussion, below), share at least 70 percent sequence identity, preferably at least 80 percent sequence identity, more preferably at least 90 percent sequence identity, and most preferably at least 95 percent amino acid identity or more. "Percentage amino acid identity" or "percentage amino acid sequence identity" refers to a comparison of the amino acids of two polypeptides which, when optimally aligned, have approximately the designated percentage of the same amino acids. For example, "95% amino acid identity" refers to a comparison of the amino acids of two polypeptides which when optimally aligned have 95% amino acid identity. Preferably, residue positions which are not identical differ by conservative amino acid substitutions. For example, the substitution of amino acids having similar chemical properties such as charge or polarity are not likely to effect the properties of a protein. Examples include glutamine for asparagine or glutamic acid for aspartic acid.

### DETAILED DESCRIPTION

This invention is the discovery that the presence of mutations in the gene designated *MEN1* are associated with individuals that are at risk for sporadic cancers, including sporadic endocrine cancers, and diseases including multiple endocrine neoplasias. These mutations can result in lack of expression of a protein, expression of a nonfunctional protein form, or significant underexpression of a functional form. Below we teach how to obtain and identify an *MEN1* gene allele. Also taught is how to express and purify the *MEN1* gene product menin, including a description of the various conventional methods one could use to detect and quantify the expression and quality of menin.

Sequencing of DNA from MEN-1 affected individuals allowed us to identify both a wild-type *MEN1* allele and many mutated forms of *MEN1*. The mutated gene can be the result of a variety of different frameshift, nonsense, missense, and/or in-frame deletion mutations; analysis of mutated forms of *MEN1* isolated from affected individuals are summarized in Figures 3 and 4.

The wild-type, functional *MEN1* gene contains 10 exons and extends across 9 kb. It appears that the *MEN1* gene is ubiquitously expressed as a 2.8 kb transcript. The predicted 610 amino acid protein product, for which we propose the name "menin", exhibits no apparent similarities with any previously known proteins.

The identification of the *MEN1* gene provides a new window into the mechanism of endocrine tumorigenesis, facilitates accurate early diagnosis of MEN 1 associated cancers, and provides preclinical identification of individuals with the FMEN1 syndrome, *i*.*e*., cancer free individuals that are at high risk of acquiring (predisposed to) *MEN1* associated tumors.

### Cloning, Analysis and Expression of the MEN1 Gene

### A. General Recombinant DNA Methods.

This invention relies on routine techniques in the field of recombinant genetics. A basic text disclosing the general methods of use in this invention is Sambrook et al., *Molecular Cloning, A Laboratory Manual*, Cold Spring Harbor Publish., Cold Spring Harbor, NY 2nd ed. (1989) and Kriegler, *Gene Transfer and Expression: A Laboratory Manual*, W.H. Freeman, N.Y., (1990). Unless otherwise stated all enzymes are used in accordance with the manufacturer's instructions.

Nucleotide sizes are given in either kilobases (Kb) or base pairs (bp). These are estimates derived from agarose or acrylamide gel electrophoresis or from published DNA sequences.

Oligonucleotides that are not commercially available can be chemically synthesized according to the solid phase phosphoramidite triester method first described by S.L. Beaucage and M.H. Caruthers, *Tetrahedron Letts*., 22(20): 1859-1862 (1981), using an automated synthesizer, as described in D.R. Needham Van Devanter *et. al*., *Nucleic Acids Res.,* 12:6159-6168, 1984. Purification of oligonucleotides is by either native acrylamide gel electrophoresis or by anion-exchange HPLC as described in J.D. Pearson and F.E. Reanier, *J*. *Chrom*., 255:137-149, 1983.

The sequence of the cloned genes and synthetic oligonucleotides can be verified using the chemical degradation method of A.M. Maxam et al., *Methods in Enzymology*, 65:499560, (1980). The sequence can be confirmed after the assembly of the oligonucleotide fragments into the double-stranded DNA sequence using the method of Maxam and Gilbert, supra, or the chain termination method for sequencing double-stranded templates of R.B. Wallace *et al*. *Gene,* 16:21-26, 1981. Southern blot hybridization techniques are carried out according to Southern *et al*., *J*. *Mol*. *Biol*., 98:503, 1975.

### B. Cloning Methods for the Isolation of Nucleotide Sequences Encoding Menin Proteins.

In general, the nucleic acid sequences encoding menin are cloned from DNA sequence libraries that are made to encode copy DNA (cDNA) or genomic DNA. The particular sequences can be located by hybridizing with an oligonucleotide probe, the sequence of which can be derived from table 1 which provides PCR primers and defines suitable regions for isolating MEN1 specific probes. Alternatively, where the sequence is cloned into an expression library, the expressed recombinant menin be detected immunologically with antisera or purified antibodies made against menin.

To make the cDNA library, one should choose a source that is rich in mRNA. For example, liver is enriched for mRNA of menin. The mRNA can then be made into cDNA, ligated into a recombinant vector, and transfected into a recombinant host for propagation, screening and cloning. Methods for making and screening cDNA libraries are well known. See Gubler, U. and Hoffman, B.J. *Gene* 25:263-269, 1983 and Sambrook.

For a genomic library, the DNA is extracted from the tissue and either mechanically sheared or enzymatically digested to yield fragments of about 12-20kb. The fragments are then separated by gradient centrifugation from undesired sizes and are constructed in bacteriophage lambda vectors. These vectors and phage are packaged *in vitro*, as described in Sambrook. Recombinant phage are analyzed by plaque hybridization as described in Benton and Davis, *Science,* 196:180-182 (1977). Colony hybridization is carried out as generally described in M. Grunstein et al. *Proc. Natl*. *Acad*. *Sci. USA*., 72:3961-3965 (1975).

An alternative method combines the use of synthetic oligonucleotide primers with polymerase extension on an mRNA or DNA template. This polymerase chain reaction (PCR) method amplifies nucleic acid sequences of MEN I directly from mRNA, from cDNA, from genomic libraries or cDNA libraries. Restriction endonuclease sites can be incorporated into the primers. Polymerase chain reaction or *other in vitro* amplification methods may also be useful, for example, to clone nucleic acid sequences that code for proteins to be expressed, to make nucleic acids to use as probes for detecting the presence of menin encoding mRNA in physiological samples, for nucleic acid sequencing, or for other purposes. U.S. Patents Nos. 4,683,195 and 4,683,202 describe this method. Genes amplified by the PCR reaction can be purified from agarose gels and cloned into an appropriate vector. Appropriate primers and probes for identifying *MEN1* genes encoding menin protein from alternative mammalian biological samples, including cells and tissues, are generated from comparisons of the sequences provided herein. For a general overview of PCR see *PCR Protocols: A Guide to Methods and Applications*. (Innis, M, Gelfand, D., Sninsky, J. and White, T., eds.), *Academic Press,* San Diego (1990).

Synthetic oligonucieotides can be used to construct *MEN1* genes. For example, this can be done using a series of overlapping oligonucleotides usually 40 to 120 bp in length, representing both the sense and nonsense strands of the gene. These DNA fragments are then annealed, ligated and cloned.

The gene for menin can be cloned using intermediate vectors before transformation into mammalian cells for expression. These intermediate vectors are typically prokaryote vectors or shuttle vectors. Menin can be expressed in either prokaryotes or eukaryotes.

In summary, the menin gene can identified and prepared by probing or amplifying select regions of a biological sample, such as a mixed cDNA or genomic pool, using the probes and primers generated from the *MEN1* sequences; exemplary probes are provided herein in Table 1 (sequence numbering based on SEQ ID NO:1):

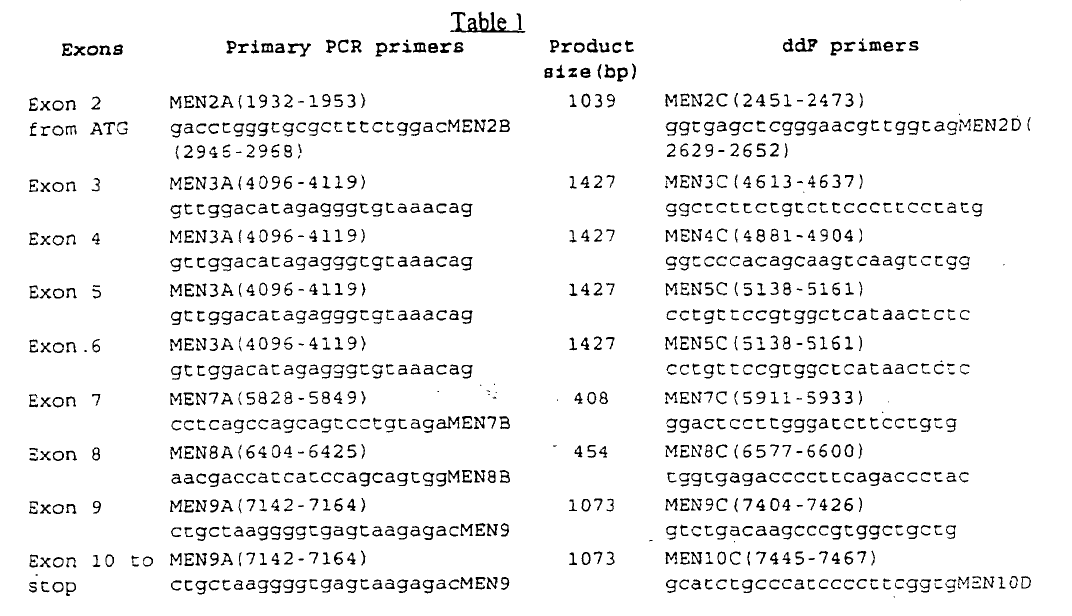

### C. Analysis of menin-encoding Nucleic Acid Sequences

The menin polypeptide encoding nucleic acid sequences of the invention includes genes and gene products identified and characterized by analysis using the sequences nucleic acid and protein sequences of the invention, including SEQ ID NO:1 and SEQ ID NO:2, respectively. Optimal alignment of sequences for comparison can use any means to analyze sequence identity (homology) known in the art, *e*.*g*., by the progressive alignment method of termed "PILEUP" (see below); by the local homology algorithm of Smith & Waterman (1981) *Adv*. *Appl*. *Math*. 2: 482; by the homology alignment algorithm of Needleman & Wunsch (1970) *J*. *Mol*. *Biol*. 48:443; by the search for similarity method of Pearson (1988) *Proc. Natl*. *Acad*. *Sci. USA* 85: 2444; by computerized implementations of these algorithms (e.g., GAP, BESTFIT, FASTA, and TFASTA in the Wisconsin Genetics Software Package, Genetics Computer Group, 575 Science Dr., Madison, WI); ClustalW (CLUSTAL in the PC/Gene program by Intelligenetics, Mountain View, CA), described by, *e*.*g*., Higgins (1988) *Gene* 73: 237-244; Corpet (1988) *Nucleic Acids Res.* 16:10881-90; Huang (1992) *Computer Applications in the Biosciences* 8:155-65, and Pearson (1994) *Methods in Molec. Biol*. 24:307-31). Other sequence alignment computer programs include Pfam (Sonnhammer (1998) *Nucleic Acids Res.* 26:322-325); TreeAlign (Hein (1994) *Methods Mol*. *Biol*. 25:349-364); NES-ALIGN, and SAM programs; or, visual inspection can identify sequence identities.

One example of a useful algorithm is PILEUP. PILEUP creates a multiple sequence alignment from a group of related sequences using progressive, pairwise alignments to show relationship and percent sequence identity. It also plots a tree or dendogram showing the clustering relationships used to create the alignment. PILEUP uses a simplification of the progressive alignment method of Feng & Doolittle (1987) *J*. *Mol*. *Evol*. 35:351-360; see also the method of Higgins & Sharp (1989) *CABIOS* 5:151-153. The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters For example, a reference sequence can be compared to other test sequences to determine the percent sequence identity relationship using the following parameters: default gap weight (3.00), default gap length weight (0.10), and weighted end gaps. See also Morrison (1997) *Mol*. *Biol*. *Evol.* 14:428-441, as an example of the use of PILEUP.

Another example of algorithm that is suitable for determining sequence similarity is the BLAST algorithm, which is described in Altschul (1990) *J*. *Mol*. *Biol*. 215: 403-410. Software for performing BLAST analyses is publicly available through the National Center for Biotechnology Information, http://www.ncbi.nlm.nih.gov/ ; see also Zhang (1997) *Genome Res.* 7:649-656 (1997) for the "PowerBLAST" variation. This algorithm involves first identifying high scoring sequence pairs (HSPs) by identifying short words of length W in the query sequence that either match or satisfy some positive-valued threshold score T when aligned with a word of the same length in a database sequence. T is referred to as the neighborhood word score threshold (Altschul (1990) *supra*). These initial neighborhood word hits act as seeds for initiating searches to find longer HSPs containing them. The word hits are extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a wordlength (W) of 11, the BLOSUM62 scoring matrix (*see* Henikoff (1992) *Proc*. *Natl*. *Acad Sci. USA* 89:10915-10919) alignments (B) of 50, expectation (E) of 10, M=5, N=-4, and a comparison of both strands. The term BLAST refers to the BLAST algorithm which performs a statistical analysis of the similarity between two sequences; *see, e.g.,* Karlin (1993) *Proc. Natl*. *Acad. Sci. USA* 90:5873-5787. One measure of similarity provided by the BLAST algorithm is the smallest sum probability (P(N)), which provides an indication of the probability by which a match between two nucleotide or amino acid sequences would occur by chance.

In addition to calculating percent sequence identity, the BLAST algorithm also performs a statistical analysis of the similarity between two sequences (*see, e.g.,* Karlin & Altschul (1993) *Proc. Natl*. *Acad*. *Sci*. *USA* 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

### D. Sequencing of Menin-Encoding Nucleic Acid

Sequencing of isolated menin-encoding nucleic acid is used, e.g., to identify and characterize wild-type allelic variations; to identify mutations found *in vivo* or generated *in vitro;* to confirm sequence of cloned or amplified nucleic acid; and the like. Menin-encoding sequences can be sequenced as inserts in vectors, as inserts released and isolated from the vectors or in any of a variety of other forms (*i*.*e*., as amplification products). Menin-encoding inserts can be released from the vectors by restriction enzymes or amplified by PCR or transcribed by a polymerase. For sequencing of the inserts to identify full length coding sequences, primers based on the N- or C- terminus, or based on insertion points in the original phage or other vector, can be used. Additional primers can be synthesized to provide overlapping sequences. Nucleic acid sequencing techniques are well known, see, *e.g*. Rosenthal (1987) *supra*; Arlinghaus (1997) *Anal. Chem.* 69:3747-3753; Dubiley (1997) *Nucleic Acids Res.* 25:2259-2265, for use of biosensor chips for identification and sequencing of nucleic acids.

### E. Expression in Prokaryotes

To obtain high level expression of a cloned gene, such as those cDNAs encoding menin in a prokaryotic system, it is essential to construct expression plasmids which contain, at the minimum, a strong promoter to direct transcription, a ribosome binding site for translational initiation, and a transcription/translation terminator. Examples of regulatory regions suitable for this purpose in *E. coli* are the promoter and operator region of the *E. coli* tryptophan biosynthetic pathway as described by Yanofsky, C., *J*. *Bacteriol*., 158:1018-1024 (1984), and the leftward promoter of phage lambda (P_{L}) as described by Herskowitz,I. and Hagen, D., *Ann. Rev. Genet.,* 14:399-445 (1980).

Expression systems for expressing the menin protein are available using a variety of bacteria including *E. coli, Bacillus sp.* and *Salmonella* (Palva (1983) *Gene* 22:229-235 (1983); Mosbach (1983) *Nature*, 302:543-545.

### F. Expression in Eukaryotes

Standard eukaryotic transfection methods are used to produce mammalian, yeast or insect cell lines which express large quantities of menin protein which are then purified using standard techniques. See, *e.g.,* Colley (1989) *J*. *Biol*. *Chem.* 264:17619-17622, and Guide to Protein Purification, in Vol. 182 of *Methods in Enzymology* (Deutscher ed., 1990). Transformations of eukaryotic cells are performed according to standard techniques as described by, *e.g.*, Morrison (1977) *J*. *Bact.,* 132:349-351; Clark-Curtiss (1983) *Methods in Enzymology* 101:347-362.

Any of the well known procedures for introducing foreign nucleotide sequences into host cells may be used. These include the use of calcium phosphate transfection, polybrene, protoplast fusion, electroporation, liposomes, microinjection, plasma vectors, viral vectors and any of the other well known methods for introducing cloned genomic DNA, cDNA, synthetic DNA or other foreign genetic material into a host cell (see Sambrook et al., *supra*). It is only necessary that the particular genetic engineering procedure utilized be capable of successfully introducing at least one gene into the host cell which is capable of expressing the menin protein.

The particular eukaryotic expression vector used to transport the genetic information into the cell is not particularly critical. Any of the conventional vectors used for expression in eukaryotic cells may be used. Expression vectors containing regulatory elements from eukaryotic viruses are typically used. SV40 vectors include pSVT7 and pMT2. Vectors derived from bovine papilloma virus include pBV-1MTHA, and vectors derived from Epstein Bar virus include pHEBO, and p205. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

The vectors of the invention can include selectable markers which result in gene amplification such as thymidine kinase, aminoglycoside phosphotransferase, hygromycin B phosphotransferase, xanthine-guanine phosphoribosyl transferase, CAD (carbamyl phosphate synthetase, aspartate transcarbamylase, and dihydroorotase), adenosine deaminase, dihydrofolate reductase, and asparagine synthetase and ouabain selection. Alternatively, high yield expression systems not involving gene amplification are also suitable, such as using a bacculovirus vector in insect cells, with a menin encoding sequence under the direction of the polyhedrin promoter or other strong baculovirus promoters.

The expression vector of the present invention can also contain both prokaryotic sequences that facilitate the cloning of the vector in bacteria as well as one or more eukaryotic transcription units that are expressed only in eukaryotic cells, such as mammalian cells. The vector may or may not comprise a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the transfected DNA integrates into the genome of the transfected cell, where the promoter directs expression of the desired gene. The expression vector is typically constructed from elements derived from different, well characterized viral or mammalian genes. For a general discussion of the expression of cloned genes in cultured mammalian cells, see, *e.g.,* Sambrook, *supra*, Ch. 16.

The prokaryotic elements that are typically included in the mammalian expression vector include a replicon that functions in *E. coli,* a gene encoding antibiotic resistance to permit selection of bacteria that harbor recombinant plasmids, and unique restriction sites in nonessential regions of the plasmid to allow insertion of eukaryotic sequences. The particular antibiotic resistance gene chosen is not critical, any of the many resistance genes known in the art are suitable. The prokaryotic sequences are preferably chosen such that they do not interfere with the replication of the DNA in eukaryotic cells.

The expression vector contains at least one eukaryotic transcription unit or expression cassette that contains all the elements required for the expression of the menin encoding DNA in eukaryotic cells. A typical expression cassette contains a promoter operably linked to the DNA sequence encoding menin and signals required for efficient polyadenylation of the transcript. Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated. Additional elements of the cassette may include enhancers and, if genomic DNA is used as the structural gene, introns with functional splice donor and acceptor sites. Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for the present invention include those derived from polyoma virus, human or murine cytomegalovirus, the long term repeat from various retroviruses such as murine leukemia virus, murine or Rous sarcoma virus and HIV. See, *e.g., Enhancers and Eukaryotic Expression,* Cold Spring Harbor Pres, Cold Spring Harbor, N.Y. 1983. In the construction of the expression cassette, the promoter is preferably positioned about the same distance from the heterologous transcription start site as it is from the transcription start site in its natural setting. As is known in the art however, some variation in this distance can be accommodated without loss of promoter function. In addition to a promoter sequence, the expression cassette should also contain a transcription termination region downstream of the structural gene to provide for efficient termination. The termination region may be obtained from the same gene as the promoter sequence or may be obtained from different genes. If the mRNA encoded by the structural gene is to be efficiently translated, polyadenylation sequences are also commonly added to the vector construct. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for the present invention include those derived from SV40, or a partial genomic copy of a gene already resident on the expression vector.

In addition to the elements already described, the expression vector of the present invention may typically contain other specialized elements intended to increase the level of expression of cloned genes or to facilitate the identification of cells that carry the transfected DNA. For instance, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The menin of the invention can also be expressed as a recombinant protein with one or more additional polypeptide domains linked thereto to facilitate protein detection, purification, or other applications. Such detection and purification facilitating domains include, but are not limited to, metal chelating peptides such as polyhistidine tracts and histidine-tryptophan modules that allow purification on immobilized metals, protein A domains that allow purification on immobilized immunoglobulin, and the domain utilized in the FLAGS extension/affinity purification system (Immunex Corp, Seattle WA). The inclusion of a cleavable linker sequences such as Factor Xa or enterokinase (Invitrogen, San Diego CA) between the purification domain and menin protein(s) may be useful to facilitate purification. An exemplary expression vector provides for expression of a menin fusion protein comprising the sequence encoding menin and nucleic acid sequence encoding six histidine residues followed by thioredoxin and an enterokinase cleavage site. The histidine residues facilitate detection and purification while the enterokinase cleavage site provides a means for purifying the desired protein(s) from the remainder of the fusion protein. Technology pertaining to vectors encoding fusion proteins and application of fusion proteins are well described in the patent and scientific literature, see *e*.*g*., Kroll (1993) DNA Cell. Biol., 12:441-53).

The DNA sequence encoding the menin can also be linked to a cleavable signal peptide sequence to promote secretion of the encoded protein by the transformed cell. Such signal peptides would include, among others, the signal peptides from tissue plasminogen activator, insulin, and neuron growth factor, and juvenile hormone esterase of *Heliothis virescens.*

### G. Expression in Cell Cultures.

Menin cDNA can be ligated to various expression vectors for use in transforming host cell cultures. The vectors typically contain gene sequences to initiate transcription and translation of the menin gene. These sequences need to be compatible with the selected host cell. In addition, the vectors preferably contain a marker to provide a phenotypic trait for selection of transformed host cells such as dihydrofolate reductase or metallothionein. Additionally, a vector might contain a replicative origin.

Cells of mammalian origin are illustrative of cell cultures useful for the production of the menin. Mammalian cell systems often will be in the form of monolayers of cells although mammalian cell suspensions may also be used. Illustrative examples of mammalian cell lines include VERO and HeLa cells, Chinese hamster ovary (CHO) cell lines, WI38, BHK, COS-7 or MDCK cell lines. NIH 3T3 or COS cells are preferred.

As indicated above, the vector, *e.g*., a plasmid, which is used to transform the host cell, preferably contains DNA sequences to initiate transcription and sequences to control the translation of the menin gene sequence. These sequences are referred to as expression control sequences. Illustrative expression control sequences are obtained from the SV-4O promoter (*Science*, 222:524-527, (1983)), the CMV I.E. Promoter (*Proc. Natl. Acad. Sci.* 81:659-663, (1984)) or the metallothionein promoter (*Nature* 296:39-42, (1982)). The cloning vector containing the expression control sequences is cleaved using restriction enzymes and adjusted in size as necessary or desirable and ligated with sequences encoding menin by means well known in the art.

As with yeast, when higher animal host cells are employed, polyadenlyation or transcription terminator sequences from known mammalian genes need to be incorporated into the vector. An example of a terminator sequence is the polyadenlyation sequence from the bovine growth hormone gene. Sequences for accurate splicing of the transcript may also be included. An example of a splicing sequence is the VP 1 intron from SV40 (Sprague, J. et al., *J*. *Virol*. 45: 773-781, (1983)).

Additionally, gene sequences to control replication in the host cell may be incorporated into the vector such as those found in bovine papilloma virus type-vectors. Saveria-Campo, M., *"Bovine Papilloma virus DNA a Eukaryotic Cloning Vector*" in DNA Cloning Vol.II a Practical Approach Ed. D.M. Glover, IRL Press, Arlington, Virginia pp. 213-238, (1985).

The transformed cells are cultured by means well known in the art. For example, as published in *Biochemical Methods in Cell Culture and Virology,* Kuchler, R.J., Dowden, Hutchinson and Ross, Inc., (1977). The expressed G-6-Pase protein is isolated from cells grown as suspensions or as monolayers. The latter are recovered by well known mechanical, chemical or enzymatic means

### 1. Expression in Yeast

Synthesis of heterologous proteins in yeast is well known and described. *Methods in Yeast Genetics,* Sherman, F., et al., Cold Spring Harbor Laboratory, (1982) is a well recognized work describing the various methods available to produce menin in yeast.

For high level expression of a gene in yeast, it is essential to connect the gene to a strong promoter system as in the prokaryote and also to provide efficient transcription termination/polyadenylation sequences from a yeast gene. Examples of useful promoters include GAL1,1O (Johnson (1984) *Mol*. *and Cell*. *Biol*. 4:1440-1448) ADH2 (Russell (1983) *J. Biol*. *Chem*. 258:2674-2682), PHO5 (*EMBO J*. 6:675-680, (1982)), and MF alpha-1. A multicopy plasmid with a selective marker such as Leu-2, URA-3, Trp-1, and His-3 is also desirable. The MF alpha-1 promoter is preferred for expression of menin in yeast. The MF alpha-1 promoter, in a host of the alpha mating-type is constitutive, but is switched off in diploids or cells with the a mating-type. It can, however, be regulated by raising or lowering the temperature in hosts which have a ts mutation at one of the SIR loci. The effect of such a mutation at 35°C on an alpha type cell is to turn on the normally silent gene coding for the alpha mating-type. The expression of the silent a mating-type gene, in turn, turns off the MF alpha-1 promoter. Lowering the temperature of growth to 27°C reverses the whole process, i.e., turns the a mating-type off and turns the MF alpha-1 on (Herskowitz, I. and Oshima, Y., in *The Molecular Biology of the Yeast Saccharomyces,* (eds. Strathern, J.N. Jones, E.W., and Broach, J.R., Cold Spring Harbor Lab., Cold Spring Harbor, N.Y., pp. 181-209, (1982). The polyadenylation sequences are provided by the 3'-end sequences of any of the highly expressed genes, like ADH1, MF alpha-1, or TPI (Alber, T. and Kawasaki, G., *J*. *of Mol*. *& Appl. Genet*. 1:419-434, (1982).

A number of yeast expression plasmids like YEp6, YEp13, YEp4 can be used as vectors. A gene of interest can be fused to any of the promoters in various yeast vectors. The above-mentioned plasmids have been fully described in the literature, e.g., Botstein (1979) *Gene* 8:17-24; Broach (1979) *Gene*, 8:121-133.

Two procedures are used in transforming yeast cells. In one case, yeast cells are first converted into protoplasts using zymolyase, lyticase or glusulase, followed by addition of DNA and polyethylene glycol (PEG). The PEG-treated protoplasts are then regenerated in a 3% agar medium under selective conditions. Details of this procedure are given in the papers by, *e*.*g*, Beggs (1978) *Nature* 275: 104-109; Hinnen (1978) *Proc. Natl*. *Acad. Sci. USA* 75:1929-1933. The second procedure does not involve removal of the cell wall. Instead the cells are treated with lithium chloride or acetate and PEG and put on selective plates (Ito, H., et al., *J*. *Bact.,* 153:163-168, (1983)).

Menin can be isolated from yeast by lysing the cells and applying standard protein isolation techniques to the lysates. The monitoring of the purification process can be accomplished by using Western blot techniques or radioimmunoassays.

### 3. Expression in insect cells

In one embodiment, recombinant menin of the invention is expressed in insect cells infected with an expression vector, such as baculovirus. The baculovirus expression vector utilizes the highly expressed and regulated *Autographa californica* nuclear polyhedrosis virus (AcMNPV) polyhedrin promoter modified for the insertion of foreign genes. Synthesis of polyhedrin protein results in the formation of occlusion bodies in the infected insect cell. The recombinant proteins expressed using this vector have been found in many cases to be, antigenically, immunogenically, and functionally similar to their natural counterparts. In addition, the baculovirus vector utilizes many of the protein modification, processing, and transport systems that occur in higher eukaryotic cells.

Briefly, the DNA sequence encoding menin is inserted into a transfer plasmid vector in the proper orientation downstream from the polyhedrin promoter, and flanked on both ends with baculovirus sequences. Cultured insect cell, commonly *Spodoptera frugiperda*, are transfected with a mixture of viral and plasmid DNAs. The virus that develop, some of which are recombinant virus that result from homologous recombination between the two DNAs, are plated at 100-1000 plaques per plate. The plaques containing recombinant virus can be identified visually because of their ability to form occlusion bodies or by DNA hybridization. The recombinant virus is isolated by plague purification. The resulting recombinant virus, capable of expressing menin, is self propagating in that no helper virus is required for maintenance or replication. After infecting an insect culture with recombinant virus, one can expect to find recombinant protein within 48-72 hours. The infection is essentially lyrtic within 4-5 days. There are a variety of transfer vectors into which the *MEN1* can be inserted. For a summary of transfer vectors.see, e.g., Luckow (1988) *Bio*/*Technology* 6:47-55. In one embodiment, the transfer vector pAcUW21 described by Bishop (1992) *Seminars in Virology* 3 :253-264, is used.

In a preferred embodiment, the insect expression system for the production of human menin utilizes *Drosophila* Schneider 2 cells (Schneider (1972) *J*. *Embryol*. *Exp*. *Morph*. 27:363-365) transfected with an appropriate vector containing the menin cDNA of the invention. In one embodiment, the pMT/V5-His vector is used; it contains an inducible metallothionein promoter for regulated transcription of insert (*i*.*e*., menin) RNA (Johansen (1989) *Genes and Develop*. 3:882-889). pMT/V5-His also contains a sequence encoding six histidines linked to the carboxy terminus of menin in order to facilitate purification of the menin protein. In various embodiments, restriction sites are added to the menin cDNA using, *e*.*g*., PCR, prior to insertion into compatable restriction sites in the selected expression vector, *e.g.,* the pMT/V5-His vector. S2 cells can be transiently or stably transfected with the pMT/V5-His menin vector. In the latter case, a selection plasmid, pCoHYGRO (van der Straten (1989) *Curr*. *Methods Mol*. *Cell. Biol* 1: 1-8), must be co-transfected into S2 cells to produce stable cells under the antibiotic selection of hygromycin-B. S2 cells are grown at 23°C in DES Expression Medium supplemented with 2mM L-glutamine. To characterize and isolate the recombinantly expressed human menin polypeptide, the cells are harvested and lysed in a buffer containing 50mM Tris HCl (pH 7.8), 150 mM NaCl and 1% Nonidet P-40 (Invitrogen Corp.,Carlsbad, CA).

Two major advantages of the insect expression systems, compared to mamalian cell expression systems, are: that the insect (e.g, *Drosophila*) system does not require isolation of clones; and, hundreds of copies of the recombinant plasmid usually become integrated into the *Drosophila* genome, resulting in higher levels of expression of the polypeptide encoded by the vector insert (Kirkpatrick (1995) *J*. *Biol*. *Chem*. 270:19800-1980).

### 3. Expression in Mammalian Cells

The invention provides for expression of *MEN1* encoding nucleic acids in mammalian cells, *in vitro* and *in vivo*, for both the production of recombinant menin and reconstitution of menin activity, for, *e.g*., experimental or therapeutic purposes. A variety of human diseases may be treated by gene therapy approaches that involve stably introducing a gene into a human cell such that the gene may be transcribed and the gene product may be produced in the cell, as discussed in detail below. *MEN1* encoding nucleic acids can be inserted any expression cassette/ expression vector using any technique known in the art; a few exemplary systems and methods are provided herein.

### Expression in recombinant vaccinia virus-infected cells

The gene encoding menin can be inserted into a plasmid designed for producing recombinant vaccinia, such as pGS62, Langford (1986) *Mol*. *Cell. Biol*. 6:3191-3199. This plasmid consists of a cloning site for insertion of foreign genes, the P7.5 promoter of vaccinia to direct synthesis of the inserted gene, and the vaccinia TK gene flanking both ends of the foreign gene. When the plasmid containing *MEN1* is constructed, the gene can be transferred to vaccinia virus by homologous recombination in the infected cell. To achieve this, suitable recipient cells are transfected with the recombinant plasmid by standard calcium phosphate precipitation techniques into cells already infected with the desirable strain of vaccinia virus, such as Wyeth, Lister, WR or Copenhagen. Homologous recombination occurs between the TK gene in the virus and the flanking TK gene sequences in the plasmid. This results in a recombinant virus with the foreign gene inserted into the viral TK gene, thus rendering the TK gene inactive. Cells containing recombinant viruses are selected by adding medium containing 5-bromodeoxyuridine, which is lethal for cells expressing a TK gene. Confirmation of production of recombinant virus can be achieved by DNA hybridization using cDNA encoding the menin protein and by immunodetection techniques using antibodies specific for the expressed protein. Virus stocks may be prepared, *e*.*g*., by infection of cells such as HeLA S3 spinner cells and harvesting of virus progeny

### Purification of Menin

A number of conventional procedures can be employed when recombinant or synthetic menin is being purified. After expression or chemical synthesis, menin may be purified to substantial purity by standard techniques, including, *e.g*.: selective precipitation with such substances as ammonium sulfate; column chromatography; immunopurification methods; and others. *See,* for instance, R. Scopes, *Protein Purification: Principles and Practice*, Springer-Verlag: New York (1982), U.S. Patent No. 4,673,641, Ausubel, and Sambrook. For example proteins having established molecular adhesion properties can be reversibly fused (chemically joined) to the menin (see discussion on fusion protein, *supra*). With the appropriate ligand, the menin can be selectively adsorbed to a purification column and then freed from the column in a relatively pure form. The fused/joined non-menin protein is then removed, *e.g*., by enzymatic activity, chemical reduction. Menin can also be purified using immunoaffinity columns.

### A. Purification of Menin from Recombinant Bacteria.

When recombinant proteins are expressed by the transformed bacteria in large amounts, typically after promoter induction; but expression can be constitutive, the proteins may form insoluble aggregates. There are several protocols that are suitable for purification of menin inclusion bodies. For example, purification of aggregate proteins (hereinafter referred to as inclusion bodies) typically involves the extraction, separation and/or purification of inclusion bodies by disruption of bacterial cells, typically but not limited by, incubation in a buffer of about 100-150 µg/mL lysozyme and 0.1% Nonidet P40, a non-ionic detergent. The cell suspension can be ground using a Polytron grinder (Brinkman Instruments, Westbury, N.Y.). Alternatively, the cells can be sonicated on ice. Alternate methods of lysing bacteria are described in Ausubel and Sambrook and will be apparent to those of skill in the art. The cell suspension is generally centrifuged and the pellet containing the inclusion bodies resuspended in buffer which does not dissolve but washes the inclusion bodies, *e.g*., 20 mM Tris-HCl (pH 7.2), 1 mM EDTA, 150 mM NaCl and 2% Triton-X 100, a non-ionic detergent. It may be necessary to repeat the wash step to remove as much cellular debris as possible. The remaining pellet of inclusion bodies may be resuspended in an appropriate buffer (e.g. 20 mM sodium phosphate, pH 6.8, 150 mM NaCl). Other appropriate buffers will be apparent to those of skill in the art. Following the washing step, the inclusion bodies are solubilized by the addition of a solvent that is both a strong hydrogen acceptor and a strong hydrogen donor (or a combination of solvents each having one of these properties); the proteins that formed the inclusion bodies may then be renatured by dilution or dialysis with a compatible buffer. Suitable solvents include, but are not limited to urea (from about 4 M to about 8 M), formamide (at least about 80%, volume/volume basis), and guanidine hydrochloride (from about 4 M to about 8 M). Some solvents which are capable of solubilizing aggregate-forming proteins, for example SDS (sodium dodecyl sulfate), 70% formic acid, are inappropriate for use in this procedure due to the possibility of irreversible denaturation of the proteins, accompanied by a lack of immunogenicity and/or activity. Although guanidine hydrochloride and similar agents are denaturants, this denaturation is not irreversible and renaturation may occur upon removal (by dialysis, for example) or dilution of the denaturant, allowing re-formation of immunologically and/or biologically active menin. After solubilization, the protein can be separated from other bacterial proteins by standard separation techniques.

Alternatively it is possible to purify menin from bacteria periplasm. Where menin protein is exported into the periplasm of the bacteria, the periplasmic fraction of the bacteria can be isolated by cold osmotic shock in addition to other methods known to skill in the art (see Ausubel). To isolate recombinant proteins from the periplasm, the bacterial cells are centrifuged to form a pellet. The pellet is resuspended in a buffer containing 20% sucrose. To lyse the cells, the bacteria are centrifuged and the pellet is resuspended in ice-cold 5 mM MgSO₄ and kept in an ice bath for approximately 10 minutes. The cell suspension is centrifuged and the supernatant decanted and saved. The recombinant proteins present in the supernatant can be separated from the host proteins by standard separation techniques well known to those of skill in the art.

### B. Standard Protein Separation Techniques For Purifying Menin.

### 1. Solubility Fractionation

Often as an initial step and if the protein mixture is complex, an initial salt fractionation can separate many of the unwanted host cell proteins (or proteins derived from the cell culture media) from the recombinant protein of interest. The preferred salt is ammonium sulfate. Ammonium sulfate precipitates proteins by effectively reducing the amount of water in the protein mixture. Proteins then precipitate on the basis of their solubility. The more hydrophobic a protein is, the more likely it is to precipitate at lower ammonium sulfate concentrations. A typical protocol is to add saturated ammonium sulfate to a protein solution so that the resultant ammonium sulfate concentration is between 20-30%. This will precipitate the most hydrophobic of proteins. The precipitate is discarded (unless the protein of interest is hydrophobic) and ammonium sulfate is added to the supernatant to a concentration known to precipitate the protein of interest. The precipitate is then solubilized in buffer and the excess salt removed if necessary, either through dialysis or diafiltration. Other methods that rely on solubility of proteins, such as cold ethanol precipitation, are well known to those of skill in the art and can be used to fractionate complex protein mixtures.

### 2. Size Differential Filtration

Menin has a calculated molecular weight of 67.5 kilodaltons and this knowledge can be used to isolated it from proteins of greater and lesser size using ultrafiltration through membranes of different pore size (for example, Amicon or Millipore membranes). As a first step, the protein mixture is ultrafiltered through a membrane with a pore size that has a lower molecular weight cut-off than the molecular weight of the protein of interest. The retentate of the ultrafiltration is then ultrafiltered against a membrane with a molecular cut off greater than the molecular weight of the protein of interest. The recombinant protein will pass through the membrane into the filtrate. The filtrate can then be chromatographed as described below.

### 3. Column Chromatography

Menin can also be separated from other proteins on the basis of their size, net surface charge, hydrophobicity and affinity for ligands. In addition, antibodies raised against proteins can be conjugated to column matrices and the proteins immunopurified. All of these methods are well known in the art. It will be apparent to one of skill that chromatographic techniques can be performed at any scale and using equipment from many different manufacturers (*e*.*g*., Pharmacia Biotech).

### Detection and Analysis of MEN1.

As should be apparent to those of skill, the invention is the identification of the *MEN1* gene, the menin polypeptide, the association of mutations in the *MEN1* gene with a predisposition to cancer and benign tumors (the heterozygote with one wild-type functional *MEN1* allele and one non-functional *MEN1* allele), and the presence of no *MEN1* allele capable of producing a functional menin polypeptide in cancerous cells (uncontrolled cell growth caused by loss of heterozygosity (LOS), *i*.*e*., loss of the one remaining wild-type functional allele). The invention is also the discovery that there are many different pedigrees of *MEN1* loss of function mutations, *i.e*., different patients suffering from multiple endocrine neoplasia type 1 (MEN1) have different mutations in their *MEN1* gene. Accordingly, the present invention also includes methods for detecting the presence, alteration or absence of *MEN1* DNA or RNA, or the presence or absence of a functional or non-functional menin polypeptide, in a physiological specimen. The methods of the invention determine the *MEN1* genotype of the individual, and thus the risk of acquiring disease associated with mutations to *MEN1*.

Although any tissue having cells bearing the genome of an individual can be used, the most convenient specimen will be blood samples or biopsies of suspect tissue bearing the germline or somatic DNA of an individual. It is also possible and preferred in some circumstances to conduct assays on cells that are isolated under microscopic visualization. A particularly useful method is the microdissection technique described in PCT application WO/95/23960. The cells isolated by microscopic visualization can be used in any of the assays described herein including both genomic and immunologic based assays

This invention provides for methods of genotyping family members in which relatives are diagnosed with multiple endocrine neoplasias type 1. Conventional methods of genotyping are provided herein. In one embodiment, genomic analysis is preferred, where mutations in *MEN1* are determined using primers and probes which bind to either the exons or introns of *MEN1* gene. A map of the *MEN1* gene is provided in Figure 1.

A variety of methods of specific DNA and RNA measurement using nucleic acid hybridization techniques are known to those of skill in the art. *See* Sambrook. For example, one method for evaluating the presence or absence of *MEN1* DNA in a sample involves a Southern transfer. Briefly, the digested genomic DNA is run on agarose slab gels in buffer and transferred to membranes. Hybridization is carried out using the probes discussed above. Visualization of the hybridized portions allows the qualitative determination of the presence, alteration or absence of *MEN1*.

Similarly, a Northern transfer may be used for the detection of menin mRNA in samples of RNA from cells expressing *MEN1*. In brief, the mRNA is isolated from a given cell sample using an acid guanidinium-phenol-chloroform extraction method. The mRNA is then electrophoresed to separate the mRNA species and the mRNA is transferred from the gel to a nitrocellulose membrane. As with the Southern blots, labeled probes are used to identify the presence or absence of the *MEN1* transcript.

The selection of a nucleic acid hybridization format is not critical. A variety of nucleic acid hybridization formats are known to those skilled in the art. For example, common formats include sandwich assays and competition or displacement assays. Hybridization techniques are generally described in *"Nucleic Acid Hybridization*, *A Practical Approach*," Ed. Hames, B.D. and Higgins, S.J., IRL Press, 1985; Gall and Pardue (1969), *Proc. Natl*. *Acad*. *Sci.,* U.S.A., 63:378-383; and John, Burnsteil and Jones (1969) *Nature*, 223:582-587. For example, sandwich assays are commercially useful hybridization assays for detecting or isolating nucleic acid sequences. Such assays utilize a "capture" nucleic acid covalently immobilized to a solid support and labeled "signal" nucleic acid in solution. The clinical sample will provide the target nucleic acid. The "capture" nucleic acid and "signal" nucleic acid probe hybridize with the target nucleic acid to form a "sandwich" hybridization complex. To be effective, the signal nucleic acid cannot hybridize with the capture nucleic acid.

It will be appreciated that nucleic acid hybridization assays can also be performed in an array-based format as described in Fodor et al. (1991) Science, 251: 767-777; Sheldon et al. (1993) Clinical Chemistry 39(4): 718-719, and Kozal et al. (1996) Nature Medicine 2(7): 753-759. In this approach, arrays bearing a multiplicity of different "probe" nucleic acids (usually amplified DNA) are hybridized against a target nucleic acid. In this manner a large number of different hybridization reactions can be run essentially "in parallel". This provides rapid, essentially simultaneous, evaluation of a wide number of reactants. Methods of performing hybridization reactions in array based formats are well known to those of skill in the art (see, e.g., Jackson et al. (1996) Nature Biotechnology, 14: 1685-1691, and Chee et al. (1995) Science, 274: 610-613).

The nucleic acid sequences used in this invention can be either positive or negative probes. Positive probes bind to their targets and the presence of duplex formation is evidence of the presence of the target. Negative probes fail to bind to the suspect target and the absence of duplex formation is evidence of the presence of the target. For example, the use of a wild type specific nucleic acid probe or PCR primers may act as a negative probe in an assay sample where only the mutant MEN1 is present.

Typically, labelled signal nucleic acids are used to detect hybridization. Complementary nucleic acids or signal nucleic acids may be labelled by any one of several methods typically used to detect the presence of hybridized polynucleotides. The most common method of detection is the use of autoradiography with ³H, ¹²⁵I, ³⁵S, ¹⁴C, or ³²P-labelled probes or the like. Other labels include ligands which bind to labelled antibodies, fluorophores, chemiluminescent agents, enzymes, and antibodies which can serve as specific binding pair members for a labelled ligand.

Detection of a hybridization complex may require the binding of a signal generating complex to a duplex of target and probe polynucleotides or nucleic acids. Typically, such binding occurs through ligand and anti-ligand interactions as between a ligand-conjugated probe and an anti-ligand conjugated with a signal. The binding of the signal generation complex is also readily amenable to accelerations by exposure to ultrasonic energy.

The label may also allow indirect detection of the hybridization complex. For example, where the label is a hapten or antigen, the sample can be detected by using antibodies. In these systems, a signal is generated by attaching fluorescent or enzyme molecules to the antibodies or in some cases, by attachment to a radioactive label. (Tijssen, P., "Practice and Theory of Enzyme Immunoassays," *Laboratory Techniques in Biochemistry and Molecular Biology*, Burdon, R.H., van Knippenberg, P.H., Eds., Elsevier (1985), pp. 9-20.)

The sensitivity of the hybridization assays may be enhanced through use of a nucleic acid amplification system which multiplies the target nucleic acid being detected. Examples of such systems include the polymerase chain reaction (PCR) system and the ligase chain reaction (LCR) system. Other methods recently described in the art are the nucleic acid sequence based amplification (NASBA™, Cangene, Mississauga, Ontario) and Q Beta Replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation.

A preferred embodiment is the use of wild-type specific or allele-specific amplifications. In the case of PCR, the amplification primers are designed to bind to a portion of the *MEN1* gene but the terminal base at the 3' end is used to discriminate between the mutant or wild-type forms of *MEN1*. If the terminal base matches the point mutation or the wild-type, polymerase dependent three prime (3'-) extension can proceed and an amplification product is detected. This method for detecting point mutations or polymorphisms is described in detail by Sommer (1989) *Mayo Clin*. *Proc.* 64:1361-1372. By using appropriate controls, one can develop a kit having both positive and negative amplification products. The products can be detected using specific probes or by simply detecting their presence or absence. A variation of the PCR method uses LCR where the point of discrimination, i.e, either the point mutation or the wild-type bases fall between the LCR oligonucleotides. The ligation of the oligonucleotides becomes the means for discriminating between the mutant and wild-type forms of *MEN1*.

An alternative means for determining the level of expression of *MEN1* is *in situ* hybridization. *In situ* hybridization assays are well known and are generally described, *e.*g., in Angerer *(1987) Methods Enzymol*. 152:649-660. In an *in situ* hybridization assay cells, preferentially bovine lymphocytes are fixed to a solid support, typically a glass slide. If DNA is to be probed, the cells are denatured with heat or alkali. The cells are then contacted with a hybridization solution at a moderate temperature to permit annealing of *MEN1* specific probes that are labelled. The probes are preferably labelled with radioisotopes or fluorescent reporters.

### Immunological Detection of Menin

In addition to the detection of *MEN1* or *MEN1* gene expression (message) using nucleic acid hybridization technology (including amplification techniques), one can also use immunoassays to detect menin polypeptide. Because menin is associated with diseases of the endocrine tissue, the determination of the presence of wild type or mutated menin is preferably performed on tissue biopsies from these organs. Immunoassays can be used to qualitatively or quantitatively analyze menin. A general overview of the applicable technology can be found in, e.g., Harlow and Lane; *Antibodies: A.Laboratory Manual*, Cold Spring Harbor Pubs., N.Y. (1988).

### A. Antibodies to Menin

Methods of producing polyclonal and monoclonal antibodies that react specifically with menin are known to those of skill in the art. *See, e.g.,* Coligan (1991), CURRENT PROTOCOLS IN IMMUNOLOGY, Wiley/Greene, NY; and Harlow and Lane; Stites *et al*. (eds.) BASIC AND CLINICAL IMMUNOLOGY (4th ed.) Lange Medical Publications, Los Altos, CA, and references cited therein; Goding (1986), MONOCLONAL ANTIBODIES: PRINCIPLES AND PRACTICE (2d ed.) Academic Press, New York, NY; and Kohler and Milstein (1975), *Nature,* 256:495-497. Such techniques include antibody preparation by selection of antibodies from libraries of recombinant antibodies in phage or similar vectors. *See,* Huse *et al*. (1989), *Science*, 246:1275-1281; and Ward *et al*. (1989), *Nature*, 341:544-546. For example, in order to produce antisera for use in an immunoassay, the menin or a antigenic fragment thereof, is isolated as described herein. For example, recombinant protein is produced in a transformed cell line. An inbred strain of mice or rabbits is immunized with the protein using a standard adjuvant, such as Freund's adjuvant, and a standard immunization protocol. Alternatively, a synthetic peptide derived from the sequences disclosed herein and conjugated to a carrier protein can be used an immunogen.

Polyclonal sera are collected and titered against the immunogen protein in an immunoassay, for example, a solid phase immunoassay with the immunogen immobilized on a solid support. Polyclonal antisera with a titer of 10⁴ or greater are selected and tested for their cross reactivity against non-menin proteins, between different wild-type menin allelic variation, or even other homologous proteins from other organisms, using a competitive binding immunoassay. Specific monoclonal and polyclonal antibodies and antisera will usually bind with a K_{D} of at least about 0.1 mM, more usually at least about 1 µM, preferably at least about 0.1 µM or better, and most preferably, 0.01 µM or better.

In producing antibodies specifically or selectively reactive with menin, a specific wild-type polymorphic variant of menin (encoded by an *MEN1* allele), or a mutated menin (the mutation typically resulting in loss of biologic activity), a recombinant protein or synthetic peptide are the preferred immunogens for the production of monoclonal or polyclonal antibodies. Naturally occurring protein may also be used either in pure or impure form. Synthetic peptides are made using the protein sequences described herein. Recombinant protein can be expressed in eukaryotic or prokaryotic cells as described above, and purified as generally described above. The product is then injected into an animal capable of producing antibodies. Either monoclonal or polyclonal antibodies may be generated, for subsequent use in immunoassays to measure the protein.

Methods of production of polyclonal antibodies are known to those of skill in the art. In brief, an immunogen, preferably a purified protein, is mixed with an adjuvant and animals are immunized. The animal's immune response to the immunogen preparation is monitored by taking test bleeds and determining the titer of reactivity to menin. When appropriately high titers of antibody to the immunogen are obtained, blood is collected from the animal and antisera are prepared. Further fractionation of the antisera to enrich for antibodies reactive to the protein can be done if desired. (*See* Harlow and Lane, *supra*).

Monoclonal antibodies may be obtained by various techniques familiar to those skilled in the art. In one exemplary method, spleen cells from an animal immunized with a desired antigen are immortalized, commonly by fusion with a myeloma cell (*See*, *e*.*g*., Kohler and Milstein, *Eur*. *J*. *Immunol*. 6:511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according to the general protocol outlined by Huse, *et al*. (1989) *Science* 246:1275-1281. Such techniques also include selection of antibodies from libraries of recombinant antibodies displayed in phage ("phage display libraries") or similar on cells. *See,* also Ward (1989) *Nature* 341:544; Hoogenboom (1997) *Trends Biotechnol.* 15:62-70; Katz (1997) *Annu*. *Rev. Biophys. Biomol*. *Struct.* 26:27-45. Recombinant antibodies can be expressed by transient or stable expression vectors in mammalian cells, as in Norderhaug (1997) *J*. *Immunol. Methods* 204:77-87;on yeast cells, as in Boder (1997) *Nat*. *Biotechnol*. 15:553-557.

Once menin specific antibodies are available, menin can be measured by a variety of immunoassay methods with qualitative and quantitative results available to the clinician. For a review of immunological and immunoassay procedures in general, see *Basic and Clinical Immunology* 7th Edition (D. Stites and A. Terr ed.) 1991. Moreover, the immunoassays of the present invention can be performed in any of several configurations, which are reviewed extensively in *Enzyme Immunoassay,* E.T. Maggio, ed., CRC Press, Boca Raton, Florida (1980); "Practice and Theory of Enzyme Immunoassays," Tijssen; and, Harlow and Lane.

Immunoassays to measure menin in a human sample may use a polyclonal antiserum which was raised to the protein completely or partially encoded by SEQ ID NO:2, or a fragment thereof. This antiserum can be selected to have low crossreactivity against non-menin proteins, or, low crossreactivity with different wild-type polymorphic variants of menin (encoded by *MEN1* alleles), or, low crossreactivity with any menin form other than that used to immunize, *i.e*, if a menin mutant polypeptide is the immunogen, low crossreactivity towards any other form of menin. Any such crossreactivity can be removed by immunoabsorption prior to use in the immunoassay.

In order to produce antisera for use in an immunoassay, menin or a fragment thereof, is isolated as described herein. For example, recombinant protein is produced in a transformed cell line. An inbred strain of mice such as Balb/c is immunized with the protein or a peptide using a standard adjuvant, such as Freund's adjuvant, and a standard mouse immunization protocol. Alternatively, a synthetic peptide derived from the sequences disclosed herein and conjugated to a carrier protein can be used an immunogen. Polyclonal sera are collected and titered against the immunogen protein in an immunoassay, for example, a solid phase immunoassay with the immunogen immobilized on a solid support. Polyclonal antisera with a titer of 10⁴ or greater are selected and tested for their cross reactivity against non-menin proteins, using a competitive binding immunoassay such as the one described in Harlow and Lane, *supra*, at pages 570-573 and below.

### B. Immunological Binding Assays.

As explained above, menin expression is associated with a normal cell phenotype and mutations that result in the under expression of menin of inability to express a functional, wild-type menin are indicative of either the existence or the likelihood of multiple endocrine neopiasia type 1. In a preferred embodiment, menin is detected and/or quantified using any of a number of well recognized immunological binding assays *(see, e.g.,* U.S. Patents 4,366,241; 4,376,110; 4,517,288; and 4,837,168). For a review of the general immunoassays, see also *Methods in Cell Biology Volume 37: Antibodies in Cell Biology,* Asai, ed. Academic Press, Inc. New York (1993); *Basic and Clinical Immunology* 7th Edition, Stites & Terr, eds. (1991). Immunological binding assays (or immunoassays) typically utilize a "capture agent" to specifically bind to and often immobilize the analyte (in this case the menin or antigenic subsequence thereof). The capture agent is a moiety that specifically binds to the analyte. In a preferred embodiment, the capture agent is an antibody that specifically binds menin (or to a specific allelic or mutant form). The antibody (anti-menin) may be produced by any of a number of means well known to those of skill in the art and as described above.

Immunoassays also often utilize a labeling agent to specifically bind to and label the binding complex formed by the capture agent and the analyte. The labeling agent may itself be one of the moieties comprising the antibody/analyte complex. Thus, the labeling agent may be a labeled menin polypeptide or a labeled anti-menin antibody. Alternatively, the labeling agent may be a third moiety, such as another antibody, that specifically binds to the antibody/menin complex. In a preferred embodiment, the labeling agent is a second menin bearing a label. Alternatively, the second antibody may lack a label, but it may, in turn, be bound by a labeled third antibody specific to antibodies of the species from which the second antibody is derived. The second can be modified with a detectable moiety, such as biotin, to which a third labeled molecule can specifically bind, such as enzyme-labeled streptavidin. Other proteins capable of specifically binding immunoglobulin constant regions, such as protein A or protein G may also be used as the label agent. These proteins are normal constituents of the cell walls of streptococcal bacteria. They exhibit a strong non-immunogenic reactivity with immunoglobulin constant regions from a variety of species (*see*, generally Kronval, *et al*. (1973) *J*. *Immunol*., 111: 1401-1406, and Akerstrom, *et al*. (1985) *J*. *Immunol*., 135: 2589-2542).

Throughout the assays, incubation and/or washing steps may be required after each combination of reagents. Incubation steps can vary from about 5 seconds to several hours, preferably from about 5 minutes to about 24 hours. However, the incubation time will depend upon the assay format, analyte, volume of solution, concentrations, and the like. Usually, the assays will be carried out at ambient temperature, although they can be conducted over a range of temperatures, such as 10°C to 40°C.

### 1. Non-Competitive Assay Formats.

Immunoassays for detecting menin in biological or tissue samples may be either competitive or noncompetitive. Noncompetitive immunoassays are assays in which the amount of captured analyte (in this case the protein) is directly measured. In one preferred "sandwich" assay, for example, the capture agent (anti-menin antibodies) can be bound directly to a solid substrate where they are immobilized. These immobilized antibodies then capture menin present in the test sample. Menin is thus immobilized is then bound by a labeling agent, such as a second menin antibody bearing a label. Alternatively, the second antibody may lack a label, but it may, in turn, be bound by a labeled third antibody specific to antibodies of the species from which the second antibody is derived. The second can be modified with a detectable moiety, such as biotin, to which a third labeled molecule can specifically bind, such as enzyme-labeled streptavidin.

### 2. Competitive Assay Formats.

In competitive assays, the amount of menin (analyte) present in the sample is measured indirectly by measuring the amount of an added (exogenous) analyte (*i.e.*, the menin) displaced (or competed away) from a capture agent (anti-menin antibody) by the analyte present in the sample. In one competitive assay, a known amount of, in this case, the menin is added to the sample and the sample is then contacted with a capture agent, in this case an antibody that specifically binds to the menin. The amount of menin bound to the antibody is inversely proportional to the concentration of menin present in the sample. In a particularly preferred embodiment, the antibody is immobilized on a solid substrate. The amount of the menin bound to the antibody may be determined either by measuring the amount of menin present in an menin/antibody complex, or alternatively by measuring the amount of remaining uncomplexed protein. The amount of menin may be detected by providing a labeled menin molecule.

A hapten inhibition assay is another preferred competitive assay. In this assay a known analyte, in this case the menin is immobilized on a solid substrate. A known amount of anti-menin antibody is added to the sample, and the sample is then contacted with the immobilized menin. In this case, the amount of anti-menin antibody bound to the immobilized menin is inversely proportional to the amount of menin present in the sample. Again the amount of immobilized antibody may be detected by detecting either the immobilized fraction of antibody or the fraction of the antibody that remains in solution. Detection may be direct where the antibody is labeled or indirect by the subsequent addition of a labeled moiety that specifically binds to the antibody as described above.

Immunoassays in the competitive binding format can be used for crossreactivity determinations. For example, the partially encoded by SEQ ID NO:2 can be immobilized to a solid support. Proteins are added to the assay which compete with the binding of the antisera to the immobilized antigen. The ability of the above proteins to compete with the binding of the antisera to the immobilized protein is compared to the menin of SEQ ID NO:2. The percent crossreactivity for the above proteins is calculated, using standard calculations. Those antisera with less than 10% crossreactivity with each of the proteins listed above are selected and pooled. The cross-reacting antibodies are optionally removed from the pooled antisera by immunoabsorption with the considered proteins, *e.g*., distantly related homologues.

The immunoabsorbed and pooled antisera are then used in a competitive binding immunoassay as described above to compare a second protein, thought to be perhaps the protein of this invention, as a wild-type polymorphic variant of menin, to the immunogen protein (*i*.*e*. menin of SEQ ID NO:2, or, a polypeptide encoded by SEQ ID NO:1). In order to make this comparison, the two proteins are each assayed at a wide range of concentrations and the amount of each protein required to inhibit 50% of the binding of the antisera to the immobilized protein is determined. If the amount of the second protein required is less than 10 times the amount of immunogen that is required (to inhibit 50% of the binding of the antisera), then the second protein is said to specifically bind to an antibody generated to the immunogen

### 3. Other Assay Formats.

In a particularly preferred embodiment, Western blot (immunoblot) analysis is used to detect and quantify the presence of menin in the sample. The technique generally comprises separating sample proteins by gel electrophoresis on the basis of molecular weight, transferring the separated proteins to a suitable solid support, (such as a nitrocellulose filter, a nylon filter, or derivatized nylon filter), and incubating the sample with the antibodies that specifically bind the menin. The anti-menin antibodies specifically bind to the menin peptides on the solid support. These antibodies may be directly labeled or alternatively may be subsequently detected using labeled antibodies (*e.*g., labeled sheep antimouse antibodies) that specifically bind to the anti-menin antibodies.

Other assay formats include liposome immunoassays (LIA), which use liposomes designed to bind specific molecules (*e.g*., antibodies) and release encapsulated reagents or markers. The released chemicals are then detected according to standard techniques *(see,* Monroe *et al*. (1986) *Amer. Clin*. *Prod. Rev.* 5:34-41).

### 4. Reduction of Non-Specific Binding.

One of skill in the art will appreciate that it is often desirable to use non-specific binding in immunoassays. Particularly, where the assay involves an antigen or antibody immobilized on a solid substrate it is desirable to minimize the amount of non-specific binding to the substrate. Means of using such non-specific binding are well known to those of skill in the art. Typically, this involves coating the substrate with a proteinaceous composition. In particular, protein compositions such as bovine serum albumin (BSA), nonfat powdered milk, and gelatin are widely used with powdered milk being most preferred.

### 5. Labels.

The particular labei or detectable group used in the assay is not a critical aspect of the invention, so long as it does not significantly interfere with the specific binding of the antibody used in the assay. The detectable group can be any material having a detectable physical or chemical property. Such detectable labels have been well-developed in the field of immunoassays and, in general, most any label useful in such methods can be applied to the present invention. Thus, a label is any composition detectable by spectroscopic, photochemical, biochemical, immunochemical, electrical, optical or chemical means. Useful labels in the present invention include magnetic beads (*e*.*g*. Dynabeads™), fluorescent dyes (*e*.*g*., fluorescein isothiocyanate, Texas red, rhodamine, and the like), radiolabels (*e*.*g*., ³H, ¹²⁵I, ³⁵S, ¹⁴C, or ³²P), enzymes (*e*.*g*., horse radish peroxidase, alkaline phosphatase and others commonly used in an ELISA), and colorimetric labels such as colloidal gold or colored glass or plastic (*e*.*g*. polystyrene, polypropylene, latex, *etc*.) beads. The label may be coupled directly or indirectly to the desired component of the assay according to methods well known in the art. As indicated above, a wide variety of labels may be used, with the choice of label depending on sensitivity required, ease of conjugation with the compound, stability requirements, available instrumentation, and disposal provisions.

Non-radioactive labels are often attached by indirect means. Generally, a ligand molecule (*e.g*., biotin) is covalently bound to the molecule. The ligand then binds to an anti-ligand (*e.g*., streptavidin) molecule which is either inherently detectable or covalently bound to a signal system, such as a detectable enzyme, a fluorescent compound, or a chemiluminescent compound. A number of ligands and anti-ligands can be used thyroxine, and cortisol, it can be used in conjunction with the labeled, naturally occurring anti-ligands. Alternatively, any haptenic or antigenic compound can be used in combination with an antibody.

The molecules can also be conjugated directly to signal generating compounds, *e.g*., by conjugation with an enzyme or fluorophore. Enzymes of interest as labels will primarily be hydrolases, particularly phosphatases, esterases and glycosidases, or oxidotases, particularly peroxidases. Fluorescent compounds include fluorescein and its derivatives, rhodamine and its derivatives, dansyl, umbelliferone, *etc*. Chemiluminescent compounds include luciferin, and 2,3-dihydrophthalazinediones, *e*.*g*., luminol. For a review of various labeling or signal producing systems which may be used, see, U.S. Patent No. 4,391,904).

Means of detecting labels are well known to those of skill in the art. Thus, for example, where the label is a radioactive label, means for detection include a scintillation counter or photographic film as in autoradiography. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Finally simple colorimetric labels may be detected simply by observing the color associated with the label. Thus, in various dipstick assays, conjugated gold often appears pink, while various conjugated beads appear the color of the bead.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simpie visual inspection.

### Gene Therapy Applications

A variety of human diseases may be treated by therapeutic approaches that involve stably introducing a gene into a human cell such that the gene may be transcribed and the gene product may be produced in the cell. Diseases amenable to treatment by this approach include inherited diseases, particularly those diseases such as MEN1, where the defect is with a single gene, *MEN1*. For discussions on the application of gene therapy towards the treatment of genetic as well as acquired diseases *See* Miller, A.D. (1992) *Nature* 357:455-460, and Mulligan, R.C. (1993) *Science* 260:926-932. For a review of gene therapy procedures, see, *e*.*g*., Zhang (1996) *Cancer Metastasis Rev.* 15:385-401; Anderson, *Science* (1992) 256: 808-813; Nabel (1993) *TIBTECH* 11: 211-217; Mitani (1993) *TIBTECH* 11: 162-166; Mulligan (1993) *Science,* 926-932; Dillon (1993) *TIBTECH* 11: 167-175; Miller (1992) *Nature* 357: 455-460; Van Brunt (1988) *Biotechnology* 6(10): 1149-1154; Vigne (1995) Restorative Neurology and Neuroscience 8: 35-36; Kremer (1995) *British Medical Bulletin* 51(1) 31-44; Haddada (1995) in *Current Topics in Microbiology and Immunology,* Doerfler and Böhm (eds) Springer-Verlag, Heidelberg Germany; and Yu (1994) *Gene Therapy*, 1:13-26.

The vectors useful in the practice of the present invention are typically derived from viral genomes. Vectors which may be employed include recombinantly modified enveloped or non-enveloped DNA and RNA viruses, preferably selected from baculoviridiae, parvoviridiae, picornoviridiae, herpesveridiae, poxviridae, adenoviridiae, or picornnaviridiae. Chimeric vectors may also be employed which exploit advantageous aspects of each of the parent vector properties, see e.g., Feng (1997) *Nature Biotechnology* 15:866-870. Such viral genomes may be modified by recombinant DNA techniques to include the tumor suppressor gene and may be engineered to be replication deficient, conditionally replicating or replication competent. In the preferred practice of the invention, the vectors are replication deficient or conditionally replicating. Preferred vectors are derived from the adenoviral, adeno-associated viral and retroviral genomes.

Conditionally replicating viral vectors are used to achieve selective expression in particular cell types while avoiding untoward broad spectrum infection. Examples of conditionally replicating vectors are described, *e.g*., in Bischoff (1996) *Science* 274:373-376; Pennisi (1996) *Science* 274:342-343; Russell (1994) *Eur*. *J*. *of Cancer* 30A(8): 1165-1171. Additionally, the viral genome may be modified to include inducible promoters which achieve replication or expression of the transgene only under certain conditions. Examples of inducible promoters are known in the scientific literature (See, e.g. Yoshida (1997) *Biochem. Biophys. Res. Comm.* 230:426-430; Iida (1996) *J*. *Virol*. 70(9):6054-6059; Hwang (1997) *J. Virol* 71(9):7128-7131; Lee (1997) *Mol*. *Cell*. *Biol*. 17:5097-5105; Dreher (1997) *J*. *Biol*. *Chem* 272; 29364-29371. The transgene may also be under control of a tissue specific promoter region allowing expression of the transgene only in particular cell types.

### Retroviral Vectors

Retroviral vectors have the ability to stably integrate the transferred gene sequences into the chromosomal DNA of the target cell. Retroviral vectors are particularly attractive because they are very efficient in stably transducing a high percentage of target cells. Accordingly most of the approved gene therapy clinical protocols use retroviral vectors. *See* Miller, A.D., (1992) *supra*. Retroviral vectors are particularly useful for modifying cells because of the high efficiency with which the retroviral vectors transduce target cells and integrate into the target cell genome. Additionally, the retroviruses harboring the retroviral vector are capable of infecting cells from a wide variety of tissues.

Retroviral vectors are produced by genetically manipulating retroviruses. Retroviruses are called RNA viruses because the viral genome is RNA. Upon infection, this genomic RNA is reverse transcribed into a DNA copy which is integrated into the chromosomal DNA of transduced cells with a high degree of stability and efficiency. The integrated DNA copy is referred to as a provirus and is inherited by daughter cells as is any other gene. The wild type retroviral genome and the proviral DNA have three genes: the *gag*, the *pol* and the *env* genes, which are flanked by two long terminal repeat (LTR) sequences. The *gag* gene encodes the internal structural (nucleocapsid) proteins; the *pol* gene encodes the RNA directed DNA polymerase (reverse transcriptase); and the *env* gene encodes viral envelope glycoproteins. The 5' and 3' LTRs serve to promote transcription and polyadenylation of virion RNAs. Adjacent to the 5' LTR are sequences necessary for reverse transcription of the genome (the tRNA primer binding site) and for efficient encapsulation of viral RNA into particles (the Psi site). See Mulligan, R.C., In: *Experimental Manipulation of Gene Expression,* M. Inouye (ed), 155-173 (1983); Mann, R., *et al*., *Cell,* 33:153-159 (1983); Cone, R.D. and R.C. Mulligan, *Proceedings of the National Academy of Sciences, U*.*S*.*A*., 81:6349-6353 (1984).

The design of retroviral vectors is well known to one of skill in the art. *See* Singer, M. and Berg, P. *supra*. In brief, if the sequences necessary for encapsidation (or packaging of retroviral RNA into infectious virions) are missing from the viral genome, the result is a *cis* acting defect which prevents encapsidation of genomic RNA. However, the resulting mutant is still capable of directing the synthesis of all virion proteins. Retroviral genomes from which these sequences have been deleted, as well as cell lines containing the mutant genome stably integrated into the chromosome are well known in the art and are used to construct retroviral vectors. Preparation of retroviral vectors and their uses are described in many publications including European Patent Application EPA 0 178 220, U.S. Patent 4,405,712, Gilboa, *Biotechniques* 4:504-512 (1986), Mann, *et al*., *Cell* 33:153-159 (1983), Cone and Mulligan, *Proc. Natl*. *Acad*. *Sci. USA* 81:6349-6353 (1984), Eglitis, M.A, *et al*. (1988) *Biotechniques* 6:608-614, Miller, A.D. *et al*. (1989) *Biotechniques* 7:981-990, Miller, A.D.(1992) *Nature, supra*, Mulligan, R.C. (1993), *supra*. and Gould, B. *et al*., and International Patent Application No. WO 92/07943 entitled "Retroviral Vectors Useful in Gene Therapy".

The retroviral vector particles are prepared by recombinantly inserting the wild type MEN1 into a retrovirus vector and packaging the vector with retroviral capsid proteins by use of a packaging cell line. The resultant retroviral vector particle is incapable of replication in the host cell and is capable of integrating into the host cell genome as a proviral sequence containing the MEN1. As a result, the patient is capable of producing menin and thus restore the cells to a normal, non-cancerous phenotype.

Packaging cell lines are used to prepare the retroviral vector particles. A packaging cell line is a genetically constructed mammalian tissue culture cell line that produces the necessary viral structural proteins required for packaging, but which is incapable of producing infectious virions. Retroviral vectors, on the other hand, lack the structural genes but have the nucleic acid sequences necessary for packaging. To prepare a packaging cell line, an infectious clone of a desired retrovirus, in which the packaging site has been deleted, is constructed. Cells comprising this construct will express all structural proteins but the introduced DNA will be incapable of being packaged. Alternatively, packaging cell lines can be produced by transforming a cell line with one or more expression plasmids encoding the appropriate core and envelope proteins. In these cells, the *gag*, *pol*, and *env* genes can be derived from the same or different retroviruses.

A number of packaging cell lines suitable for the present invention are also available in the prior art. Examples of these cell lines include Crip, GPE86, PA317 and PGI3. *See* Miller *et al*., *J*. *Virol.* 65:2220-2224 (1991). Examples of other packaging cell lines are described in Cone, R. and Mulligan, R.C., *Proceedings of the National Academy of Sciences, USA,* 81:6349-6353 (1984) and in Danos, O. and R.C. Mulligan, *Proceedings of* *the National Academy of Sciences*, *USA*, 85: 6460-6464 (1988), Eglitis, M.A., et al. (1988) *supra* and Miller, A.D., (1990) *supra*. Packaging cell lines capable of producing retroviral vector particles with chimeric envelope proteins may be used. Alternatively, amphotropic or xenotropic envelope proteins, such as those produced by PA317 and GPX packaging cell lines may be used to package the retroviral vectors.

### Adeno-Associated Virus (AA V)-Based Vectors

Adeno-associated virus (AAV)-based vectors are also used to transduce cells with target nucleic acids, *e*.*g*., in the *in vitro* production of nucleic acids and peptides, and in *in vivo* and *ex vivo* gene therapy procedures. See, Okada (1996) *Gene Ther.* 3:957-964; West (1987) *Virology* 160:38-47; Carter (1989) U.S. Patent No. 4,797,368; Carter *et al*. WO 93/24641 (1993); Kotin (1994) *Human Gene Therapy* 5:793-801; Muzyczka (1994) *J*. *Clin. Invst*. 94:1351, for an overview of AAV vectors. Construction of recombinant AAV vectors are described in a number of publications, including Lebkowski, U.S. Pat. No. 5,173,414; Tratschin (1985) *Mol*. *Cell*. *Biol*. 5(11):3251-3260; Tratschin (1984) *Mol*. *Cell*. *Biol*. *4:* 2072-2081; Hermonat (1984) *Proc. Natl*. *Acad*. *Sci. USA* 81: 6466-6470; McLaughlin (1988) and Samulski (1989) *J. Virol*., 63:03822-3828. Cell lines that can be transformed by rAAV include those described in Lebkowski (1988) *Mol*. *Cell*. *Biol*., 8:3988-3996.

### Adenoviral Vectors

The invention also provides for *in vivo* expression of menin-encoding nucleic acid in an adenoviral vector suitable for gene therapy. Human clinical studies have demonstrated that adenovirus vectors are effective means to infect tumors and express transgene and viral products by tumor cells, resulting in a significant anti-tumor immune response (see, *e*.*g*., Gahery-Segard (1997) J. Clin. Invest. 100:2218-2226). The use of adenoviral vectors *in vivo,* and for gene therapy, is well described in the patent and scientific literature, *e.g.*, see, Horellou (1997) *Mol*. *Neurobiol.* 15: 241-256; Hermens (1997) *J. Neurosci*. *Methods*., Jan., 71(1): 85-98; Zeiger (1996) *Surgery* 120:921-925; Channon (1996) *Cardiovasc Res.* 32:962-972; Huang (1996) *Gene Ther*. 3:980-987; Zepeda (1996) *Gene Ther*. 3:973-979; Yang (1996) *Hum*. *Mol*. *Genet*. 5:1703-1712; Caruso (1996) *Proc. Natl*. *Acad*. *Sci. USA* 93:11302-11306; Rothmann (1996) *Gene Ther*. 3:919-926; Haecker (1996) *Hum. Gene Ther*. 7:1907-1914. The use of adenoviral vectors is described in detail in WO 96/25507. Adenovirus type 5 and adenovirus type 2 genomes are described, *e*.*g*., by Chroboczek (1992) *Virology* 186:280-285. Adenovirus vectors for gene therapy are typically made replication defective by deletion of adenovirus early ("E") region 1 ("E1") region genes.

For isolation, propagation, and large-scale production of such vectors, E 1 functions are supplied in *trans* from a stable cell line. Typically, adenovirus vectors used for clinical studies are produced in the 293 cell, a human embryonic kidney cell line expressing E1 functions from an integrated segment of the left end of the adenovirus (Ad) type 5 (Ad5) genome (see, *e.g.,* Hehir (1996) *J*. *Virol.* 70:8459-8467) (other cell lines can be used to the propagation of early region 1-deleted adenoviral vectors, e.g., the human embryonic retinoblast (HER) line 911, with a plasmid containing base pairs 79-5789 of the Ad5 genome, see Fallaux (1996) *Hum. Gene Ther*. 7:215-222). Many adenoviral vectors are engineered such that the inserted gene of interest (*e*.*g*., a transgene, the wild type menin) replaces the adenovirus E1a, E1b, and E3 genes; subsequently the replication-defective vector can be propagated only in human 293 cells that supply the deleted E1 gene functions in *trans*. Alternatively, adenoviral E1, E3, and the E2b gene functions are deleted (see, *e*.*g*., Amalfitano (1998) *J*. *Virol.* 72:926-933); or, the E1 and/or E4 genes are deleted (see, *e.g.,* Brough (1996) *J*. *Virol*. 70:6497-6501; Armentano (1995) *Hum. Gene Ther*. 6:1343-1353). Adenoviral vectors can also contain a deletion in the adenovirus early region 3 and/or early region 4.

Alternatively, to achieve amplification of recombinant replication defective adenoviral transgene expression *in vivo,* a the *trans* complementation approach can be used, where cotransduction of an E1-defective adenovirus with a plasmid containing the deleted E1 functions into cells results in E1-defective virus production by those cells (see, *e*.*g*., Dion (1996) *Cancer Gene Ther*. 3:230-237, Goldsmith (1994) *Hum*. *Gene Ther*. 5:1341-134). Adenoviral vectors can include a deletion of some or all of the protein IX gene (four trimers of Ad protein IX are embedded in the upper surface of viral hexons to create a highly-stable assembly, see, *e*.*g*., Babiss (1991) *J. Virol*. 65:598-605; Furcinitti (1989) *EMBO J*. 8:3563-3570) (for Ad pIX deletion vectors, see, *e*.*g*., Krougliak (1995) *Hum. Gene Ther*. 6:1575-1586). In one embodiment, the adenoviral vectors include deletions of the E1a and/or E 1b sequences. The following examples provide by way of illustration only and not by way of limitation. Those of skill will readily recognize a variety of noncritical parameters which could be changed or modified to yield essentially similar results.

### EXAMPLES

### Example 1: Identification of MEN1 mutations

The following example details the characterization and definition of a number of *MEN1* mutations.

Using the above methods, it was possible to define a number of *MEN1* mutations. The invention provides for the discovery that mutations in *MEN1* were responsible for multiple endocrine neoplasia type 1 (MEN1), and that some non-hereditary (*i*.*e*., somatic or acquired) mutations in both alleles of *MEN1* can be responsible for many different endocrine tumors.

Patients clinically diagnosed as having multiple endocrine neoplasia type 1 (MEN1) were selected for the study. One population studied came from 15 typical multiple endocrine neoplasia type 1 affected families. The diagnosis of MEN1 was based upon presence of tumors in two of the three principal systems (parathyroid, enteropancreatic endocrine tissue, anterior pituitary) Diagnosis of familial MEN1 required at least one first-degree relative with a tumor of one or more of these systems. There were I to 47 living affected members in each kindred, with a median of 5. All participating family members gave full informed consent in a protocol approved by the NIDDK Institutional Review Board.

Genomic DNA was isolated from blood samples using the Qiagen Kit (Chatsworth, CA). Exons 2-10 of *MEN1* were amplified individually or in groups from genomic DNA using primers designed from intron sequences. The relevant PCR primers and ddF primer sequences can be found in Table 1. PCR was performed in 25 µl reactions containing 100ng DNA, 0.2 µM of each primer, 200 µM dNTPs, 10mM Tris-HCl pH 8.3, 50mM KCI, 1.5 mM MgCl2, 0.001% (w/v) gelatin, and 0.5U AmpliTaq Gold (Perkin Elmer, Branchburg, NJ). Thermal cycling in a Perkin Elmer System 9600 was performed at 92°C for 10 minutes and then 35 cycles of 94°C for 30 sec, 60°C for 30 sec, 72°C for 1-2 min, followed by a final extension at 72°C for 5 min. DMSO at a final concentration of 5% was included for exons 2, 9 and 10.

After checking for purity of the PCR product by electrophoresing 5µl on agarose, dideoxy fingerprinting (ddF) was carried out as described by Sarkar (1992) *Genomics* 13:441, with modifications. The primary PCR products were subjected to a dideoxy chain termination reaction using ddGTP (Boehringer Mannheim, Indianapolis, IN) in a 10µl reaction containing 1µl primary PCR template (20 ng), 0.15µM end-labeled ddF primer (using gamma-³³ P ATP from Dupont-NEN, Boston, MA and T4 polynucleotide kinase from Promega, Madison, WI), 25 µM dNTPs, 200 µM ddGTP, 10 mM Tris-HCl pH 8.3, 50 mM KCI, 1.5 mM MgCl2, 0.001% (w/v) gelatin, and 1 U Amplitaq Gold. Reactions were cycled in a Perkin Elmer System 9600: 92°C for 10 min to activate Taq Gold and then 35 cycles of 94°C for 30 seconds, 65°C for 30 seconds and 72°C for 1 minute; after a final extension step at 72°C for 5 minutes the reactions were held at 4°C. To the 10 µl ddF reactions was added 40 µl of buffer containing 7M urea, 50% formamide, bromophenol blue and xylene cyanol. Reactions were heated at 94°C for 5 minutes and chilled in ice, 5µl was loaded on a non-denaturing gel (0.75X MDE, FMC Bioproducts, Rockland, ME) in 0.5X TBE) on a sequencing apparatus. The gel was electrophoresed at a constant power of 8 Watts at room temperature in a buffer system consisting of 0.5X TBE in the top reservoir and 0.8X TBE with 0.5M sodium acetate in the bottom reservoir, until the bromophenol blue reached the bottom of the gel. The gel was removed on Whatman paper and dried for 30 minutes in a sequencing gel drier and autoradiographed overnight.

One ddF primer could screen about 250 bp region; if the region to be screened in the primary PCR product was larger, additional primers were used for ddF. Samples showing changes in band patterns were subjected to cycle sequencing using the same primary PCR product and the same end-labeled primer as was used in the ddF reaction. For insertion or deletion type changes in which the actual bases involved could not be ascertained from the sequence of the heterozygous patient sample, the primary PCR product was cloned in the TA cloning vector pCRII (Invitrogen, San Diego, CA) and then sequenced.

Confirmation that the mutation segregated with *MEN1* was achieved by direct sequencing of PCR products from other affected family members. Independent confirmation of the sequence change in affected individuals was achieved by restriction digestion of the appropriate exon PCR product for 512delC (creates an *AflII* site), W436R (creates *MspI* and *NciI* sites), and R527X (creates a *Bsu361* site). For the remainder, analysis was carried out with radiolabeled allele-specific 16 to 20-mers corresponding to the wild type or mutant sequence, hybridized to slot blots of exon PCR products, as described by Lyons (1990) Science 249:655).

For example, Figure 2 shows the detection of frameshift and nonsense *MEN1* mutations. Analysis of exon 2 in an MEN1 patient and a normal control, using dideoxy fingerprinting (ddF), revealed pattern differences (arrows, Figure 2A) indicative of a *MEN1* mutation. Abnormal ddF pattern in exon 9 from a different patient is shown in Figure 2B. Identification of a single nucleotide deletion by sequencing of a cloned exon 2 PCR product from the patient whose ddF pattern is shown in Figure 2A is shown in Figure 2C. The sequence shown is of the antisense strand; the mutation is 512delC. This frameshift mutation was confirmed by detecting the presence of a new *AflII* site in PCR-amplified exon 2 from this patient and two affected relatives (Figure 2D). Direct sequencing of the exon 9 PCR product from panel (B), revealed the presence of a heterozygous C to T (C => T) substitution (Figure 2D). Again the sequence is of the antisense strand; the mutation creates a stop codon: TGG to TAG or W436X (TGG => TAG or W436X) (abbreviations indicating mutations follow standard nomenclature, see Beaudet (1993) *supra*).

Figure 3 show a summary of mutations identified in 15 unrelated MEN 1 patients. The locations of the five frameshift mutations are shown above a diagram of the *MEN1* gene, with the exons numbered; crosshatched areas are untranslated. Two in-frame deletions of a single amino acid, three nonsense mutations, and one missense mutation (W436R) are shown below the gene diagram. The 416delC and 512delC mutations were each encountered twice.

Figure 4 shows a summary of mutations identified in unrelated *MEN1* patients using similar techniques (abbreviations indicating *MEN1* mutations follow standard nomenclature, see Beaudet (1993) *supra*).

Two examples of abnormal ddF patterns are shown in Figure 2A (exon 2) and 3B (exon 9). Sequencing of PCR-amplified material (Fig. 2E), or in some instances cloned products (Fig. 2C), was used to identify the nature of the abnormality. For nine different mutations where other affected family members were available for study (all except E363del and W436X), confirmation that the observed alteration was inherited concordantly with the *MEN1* phenotype was carried out (Fig. 2D). A total of 5 frameshift mutations, 3 nonsense mutations, 2 in-frame deletions, and one missense alteration were identified (Fig. 3). Two mutations (416delC and 512delC) were encountered twice in families not known to be related. The missense mutation (W436R) was not observed in an analysis of 71 normal DNA samples. Three relatively common polymorphisms, L432L (CTG/CTA), D418D (GAC/GAT) and A541T (GCA/ACA) were also encountered, and were observed in 0.7%, 42% and 4% of normal chromosomes respectively (n=142).

The identification of significant mutations in 13 of 15 unrelated affected individuals establishes that the gene responsible for multiple endocrine neoplasia type 1 (MEN1) has been identified. The observation that many of the mutations detected (Fig. 3) would most likely result in ioss of function of the protein product is consistent with a tumor suppressor mechanism, and distinguishes MEN1 from the related disorder multiple endocrine neoplasia type 2.

### Example 2: Expression of Recombinant Human MEN1 Gene Product

The following example details the expression, isolation and characterization of recombinant human menin. The example also illustrates the production of antiserum to human menin polypeptide and its use in the characterization of menin using Western blots.

The insect expression system for the production of human menin utilizes *Drosophila* Schneider 2 cells (Schneider (1972) *J*. *Embryol*. *Exp. Morph*. 27:363-365) transfected with an appropriate vector containing the menin cDNA of the invention. The pMT/V5-His vector was used; it contains an inducible metallothionein promoter for regulated transcription of insert (*i.e*., menin) RNA (Johansen (1989) *supra*). pMT/V5-His also contains a sequence encoding six histidines linked to the carboxy terminus of menin in order to facilitate purification of the menin protein. Restriction sites were added to the menin cDNA using PCR prior to facilitate its insertion into the appropriate site in pMT/V5-His. S2 cells were stably transfected with the pMT/V5-His menin vector. The selection plasmid, pCoHYGRO (van der Straten (1989) *supra*) was co-transfected into S2 cells to produce stable cells under the antibiotic selection of hygromycin-B. S2 cells were grown at 23°C in DES Expression Medium supplemented with 2mM L-glutamine.

To characterize and isolate the recombinantly expressed human menin polypeptide, the cells were harvested and lysed in a buffer containing 50mM Tris HCl (pH 7.8), 150 mM NaCl and 1% Nonidet P-40 (Invitrogen Corp.,Carlsbad, CA).

The molecular weight of menin from was measured by SDS-PAGE under reduced conditions followed by Western blot analysis of the gel. Antiserum specific for human menin was generated by injecting a menin peptide corresponding to amino acids numbered 583 to 610 into rabbits; and purified as described by Goldsmith (1987) *J*. *Biol*. *Chem*. 262: 14683-14688. Western blot analysis with anti-menin peptide antiserum demonstrated that the recombinant menin had an apparent molecular weight of 68,000 daltons.

### SEQUENCE LISTING

### SEQ ID NO:1

X-Sun-Data-Type: text
X-Sun-Data-Description: text
X-Sun-Data-Name: text
X-Sun-Charset: us-ascii
X-Sun-Content-Lines: 11

menin.cDNA 1.28, 2772 bases

1 GGTGTCCGGA GCCGCGGACC TAGAGATCCC AGAAGCCACA GCGCAGCGGC
51 CCGGCCCGCC ACTATTTCCA GGCTCTGCGG GGCAGGGGCC GCCGCCCACC
101 GCCCGCCGCC ATGGGGCTGA AGGCCGCCCA GAAGACGCTG TTCCCGCTGC
151 GCTCCATCGA CGACGTGGTG CGCCTGTTTG CTGCCGAGCT GGGCCGAGAG
201 GAGCCGGACC TGGTGCTCCT TTCCTTGGTG CTGGGCTTCG TGGAGCATTT
251 TCTGGCTGTC AACCGCGTCA TCCCTACCAA CGTTCCCGAG CTCACCTTCC
301 AGCCCAGCCC CGCCCCCGAC CCGCCTGGCG GCCTCACCTA CTTTCCCGTG
351 GCCGACCTGT CTATCATCGC CGCCCTCTAT GCCCGCTTCA CCGCCCAGAT
401 CCGAGGCGCC GTCGACCTGT CCCTCTATCC TCGAGAAGGG GGTGTCTCCA
451 GCCGTGAGCT GGTGAAGAAG GTCTCCGATG TCATATGGAA CAGCCTCAGC
501 CGCTCCTACT TCAAGGATCG GGCCCACATC CAGTCCCTCT TCAGCTTCAT
551 CACAGGCACC AAATTGGACA GCTCCGGTGT GGCCTTTGCT GTGGTTGGGG
601 CCTGCCAGGC CCTGGGTCTC CGGGATGTCC ACCTCGCCCT GTCTGAGGAT
651 CATGCCTGGG TAGTGTTTGG GCCCAATGGG GAGCAGACAG CTGAGGTCAC
701 CTGGCACGGC AAGGGCAACG AGGACCGCAG GGGCCAGACA GTCAATGCCG
751 GTGTGGCTGA GCGGAGCTGG CTGTACCTGA AAGGATCATA CATGCGCTGT
801 GACCGCAAGA TGGAGGTGGC GTTCATGGTG TGTGCCATCA ACCCTTCCAT
851 TGACCTGCAC ACCGACTCGC TGGAGCTTCT GCAGCTGCAG CAGAAGCTGC
901 TCTGGCTGCT CTATGACCTG GGACATCTGG AAAGGTACCC CATGGCCTTA
951 GGGAACCTGG CAGATCTAGA GGAGCTGGAG CCCACCCCTG GCCGGCCAGA
1001 CCCACTCACC CTCTACCACA AGGGCATTGC CTCAGCCAAG ACCTACTATC
1051 GGGATGAACA CATCTACCCC TACATGTACC TGGCTGGCTA CCACTGTCGC
1101 AACCGCAATG TGCGGGAAGC CCTGCAGGCC TGGGCGGACA CGGCCACTGT
1151 CATCCAGGAC TACAACTACT GCCGGGAAGA CGAGGAGATC TACAAGGAGT
1201 TCTTTGAAGT AGCCAATGAT GTCATCCCCA ACCTGCTGAA GGAGGCAGCC
1251 AGCTTGCTGG AGGCGGGCGA GGAGCGGCCG GGGGAGCAAA GCCAGGGCAC
1301 CCAGAGCCAA GGTTCCGCCC TCCAGGACCC TGAGTGCTTC GCCCACCTGC
1351 TGCGATTCTA CGACGGCATC TGCAAATGGG AGGAGGGCAG TCCCACGCCT

1401 GTGCTGCACG TGGGCTGGGC CACCTTTCTT GTGCAGTCCC TAGGCCGTTT
1451 TGAGGGACAG GTGCGGCAGA AGGTGCGCAT AGTGAGCCGA GAGGCCGAGG
1501 CGGCCGAGGC CGAGGAGCCG TGGGGCGAGG AAGCCCGGGA AGGCCGGCGG
1551 CGGGGCCCAC GGCGGGAGTC CAAGCCAGAG GAGCCCCCGC CGCCCAAGAA
1601 GCCAGCACTG GACAAGGGCC TGGGCACCGG CCAGGGTGCA GTGTCAGGAC
1651 CCCCCCGGAA GCCTCCTGGG ACTGTCGCTG GCACAGCCCG AGGCCCTGAA
1701 GGTGGCAGCA CGGCTCAGGT GCCAGCACCC GCAGCATCAC CACCGCCGGA
1751 GGGTCCAGTG CTCACTTTCC AGAGTGAGAA GATGAAGGGC ATGAAGGAGC
1801 TGCTGGTGGC CACCAAGATC AACTCGAGCG CCATCAAGCT GCAACTCACG
1851 GCACAGTCGC AAGTGCAGAT GAAGAAGCAG AAAGTGTCCA CCCCTAGTGA
1901 CTACACTCTG TCTTTCCTCA AGCGGCAGCG CAAAGGCCTC TGAACTACTG
1951 GGGACTTCGG ACCGCTTGTG GGGACCCAGG CTCCGCCTTA GTCCCCCAAC
2001 TCTGAGCCCA TGTTCTGCCC CCAGCCCAAA GGGGACAGGC CTCACCTCTA
2051 CCCAAACCCT AGGTTCCCGG TCCCGAGTAC AGTCTGTATC AAACCCACGA
2101 TTTTCTCCAG CTCAGAACCC AGGGCTCTGC CCCAGTCGTT AGAATATAGG
2151 TCTCTTCTCC CAGAATCCCA GCCGGCCAAT GGAAACCTCA CGCTGGGTCC
2201 TAATTACCAG TCTTTAAAGG CCCAGCCCCT AGAAACCCAA GCTCCTCCTC
2251 GGAACCGCTC ACCTAGAGCC AGACCAACGT TACTCAGGGC TCCTCCCAGC
2301 TTGTAGGAGC TGAGGTTTCA CCCTTAACCC AAGGGAGCAC AGGTCCCACC
2351 TCCAGCCCGG GGAGCCTAGG ACCACTCAGC CCCTAGGAGT ATATTTCCGC
2401 ACTTCAGAAT TCCATATCTT GCGAATCCAA GCTCCCTGCC CCAAATAACT
2451 TCAGTCCTGC TTCCAGAATT TGGAAATCCT AGTTTCCTCT CCTTCGTATC
2501 CCGAGTCTGG GACACAAAAC TCCGCCCCCA GCCTATGAGC ATCCTGAGCC
2551 CCGCCCTCTT CCTGACGAAA CTGGCCCCGG ATCAGAGCAG GACCTCCCTT
2601 CCGACCCTCT GGGAACCTCC CAGAGGTCCA GCCCATCTCG GAGCATCCCG
2651 GAGGAAATCT GCAGAGGGGT TAGGAGTGGG TGACAAGAGC CTGATCTCTT
2701 CCTGTTTTGT ACATAGATTT ATTTTTCAGT TCCAAGAAAG ATGAATACAT
2751 TTTGTTAAAA AAAAAAAAAA AA

### SEQ ID NO:2

Mu.prot sequence 610 amino acids

1 MGLKAAQKTL FPLRSIDDVV RLFAAELGRE EPDLVLLSLV LGFVEHFLAV
51 NRVIPTNVPE LTFQPSPAPD PPGGLTYFPV ADLSIIAALY ARFTAQIRGA
101 VDLSLYPREG GVSSRELVKK VSDVIWNSLS RSYFKDRAHI QSLFSFITGT
151 KLDSSGVAFA VVGACQALGL RDVHLALSED HAWVVFGPNG EQTAEVTWHG
201 KGNEDRRGQT VNAGVAERSW LYLKGSYMRC DRKMEVAFMV CAINPSIDLH
251 TDSLELLQLQ QKLLWLLYDL GHLERYPMAL GNLADLEELE PTPGRPDPLT
301 LYHKGIASAK TYYRDEHIYP YMYLAGYHCR NRNVREALQA WADTATVIQD

351 YNYCREDEEI YKEFFEVAND VIPNLLKEAA SLLEAGEERP GEQSQGTQSQ
401 GSALQDPECF AHLLRFYDGI CKWEEGSPTP VLHVGWATFL VQSLGRFEGQ
451 VRQKVRIVSR EAEAAEAEEP WGEEAREGRR RGPRRESKPE EPPPPKKPAL
501 DKGLGTGQGA VSGPPRKPPG TVAGTARGPE GGSTAQVPAP AASPPPEGPV
551 LTFQSEKMKG MKELLVATKI NSSAIKLQLT AQSQVQMKKQ KVSTPSDYTL
601 SFLKRQRKGL

### SEQ ID NO:3

The genomic sequence for the *MEN1* gene
mu.benomic.fa 9180 bp

1 CTGGTCTTGA ACTCCTGGCC TCAAGCAATC CTCCTGCTTC AGCTTCCCAA
51 AGTGTTGTAA TTACAGGCAT GAGCCTGGCA TGAACTTGAC ACTATTGAGA
101 TATACTGGTC AGGTATTTTG TGGAATGTCC CTCAACTCTG TTTTGCCAGA
151 TGTTTTCTCA TGATTAGAGG AGAGTTATAA ATTTTGAGGA AAATCCAGAG
201 AGGTGAAGAG GTGAAGTAGG GCAGAAATTT AATCTGTTTT ATTTACTGCT
251 ATATACCGAG TGTCTGGAAC TTGGCCCATG GTAAGTACCA AAAATCTGTT
301 TTTTTTGAAT GAATAAGCAA ATAAATGAGT GACCGTGGAA ATTTAGTATT
351 ATTTCAAAGT TTCAAAGCGT TGTTGATACA GGCCAGGCAC AGTGGCTCAC
401 ACCTGTAATC CCAGCACTTT TGGAGGCCGA GGTAGGAGGA TCACTTGAGG
451 TCAGGAGTTC GAGACCAGCC TGACCAACAT GGTGACACCC CTGTCTCTAC
501 TAAGTAAAAT ACAAAAATTA GCCAAGTGTG GTGGCAGGCA CCTGTAATCC
551 CGGCTACTTG GGAAGCTGAG GCAGAAGAAT CACTTGAACC TGGGAGGCAG
601 AGGTTGCAGT GAGCCGAGAT CACCCCACTG CACTCCAGCC TGAGTGACAG
651 AGCGAGACTC TGTCTCAAAA CAAATAAACA AATAACTACT CTTTGGCCGG
701 GTAAGGTGGT TCACGCCTGT AATTTTAGCA CTTTGGGAGG CTGAGGCGGG
751 CAGATCACTT GAGGTTAGGG GTTCGAGACC AGTCTGGCCA ACATGGTGAA
801 ACCCCATCTC TACTTAAAAT ACAAAAAGTT TTCTGGGTGT GGTGGCGGAC
851 GCCTATAATC CCAGCTACTT GGGACTTTTT TTTAAGACGG AATCTCACTC
901 TGTTGCCCAG GCTGGAGTGC AGTGGCAAGA TTCTGGCTCA CTGAAGCCTC
951 CGCCTCCCAG GTTCAAGGGG ATTCCCCGCG CCTCAGCCTC CCAAGTAGCT
1001 GGGAATCCCT GTCTCTGCAA AAAAAAAAAA AAAAAAAAAC AAAAAATATA
1051 TATATATATA TATATGTGTG TGTGTGTGTG TGTGTGTGTG TTATATGTAT
1101 ATATATTTAT GTATATGCAC ATACACACAA AATTAGGCGG GAGTGGTGGC
1151 GCACGCCTGT GATCACAGCT ACTCGGGAGG CTGAGGCACG AGAATCGCTT
1201 GAGCCCGTGA AGTCGAGGCT GCAGTGAGCC CAGATCGAGC CACTGCATTC
1251 CAGCCTGGGC GAAAGAGAAA GACCGTGTCT CAAAACAAAC AAACAAAAGC
1301 TACTCTTAGC ACGTGTTAGA GTATCTCGCG GGCGGAAGTG GGAAACGAGT
1351 GCTGCACACA GAGTAGGCAT CTTTATATGT TAACAGACAC TGATACCCAG

1401 CTAAAGCGGC TGAACACATT TACTCTCTGG CAGTGTTTAA AAGTATCTGT
1451 TTTTCTCATA TTGTTTTATT TTAATTTTTT CTGGATCAAG CAACCTGATC
1501 TTTTTCCTCA TAACTTGCCG ACCGACCCGT GACAGCAAAA CCGGCAGAAG
1551 CTCGGCGACC TCCCACCCCG AGTCTGCAGG TAGTGCCCCC GGACTACATT
1601 TTCCAGAAGG CACTGCGGGC ACGCTTCCTG CCTGGTCGGC CTGAAGGGAA
1651 GGGCCAATCC CTGAGTATCT CGGGAAGGAG GTGTCCGGAG CCGCGGACCT
1701 AGAGATCCCA GAAGCCACAG CGCAGCGGCC CGGCCCGCCA CTATTTCCAG
1751 GCTCTGCGGG GCAGGGGTGG GCCCAGACTC CACTTCCCGG CGGGTAGTGC
1801 GACCCTAGGG GCGGGACTTC ATGTCCCAGC AGGCTCCGGG CGGCGTGCGC
1851 CGCGGTGCCT AGTGTGGGAT GTAAGCGCGG AGGTGGGCGA GGGGGACCGA
1901 GGCCAGGACT CTCCTTGGGG TTTGGGGGCT TGACCTGGGT GCGCTTTCTG
1951 GACAGACTTT ACAGCCCCCG GGGGCACAGT CGTAGAGAGG GGGCGGGGCG
2001 GCCATTGGGG CTCCTCATTG GGGTGCTTGG GGCGCACCCC ATCGGGTACC
2051 GGGCGTCCCG GAATTGTGGG GGACAAAAAG GCTCTGCAGT CTCGGCTGAG
2101 GGGTCTCACC GACAAAAGAG GGGAAGCCGG TGAGCAGAGG CTGAAGAGGG
2151 TGGGGAAGCA GGGGAGCTGT GCGTGTGTCG GGGCGGGTGG AACCTTAGCG
2201 GACCCTGGGA GGAGGCTCCC CGGCCGAACC TGCCCGACCC TCCCTCCCCC
2251 GGCTTGCCTT GCAGGCCGCC GCCCACCGCC CGCCGCCATG GGGCTGAAGG
2301 CCGCCCAGAA GACGCTGTTC CCGCTGCGCT CCATCGACGA CGTGGTGCGC
2351 CTGTTTGCTG CCGAGCTGGG CCGAGAGGAG CCGGACCTGG TGCTCCTTTC
2401 CTTGGTGCTG GGCTTCGTGG AGCATTTTCT GGCTGTCAAC CGCGTCATCC
2451 CTACCAACGT TCCCGAGCTC ACCTTCCAGC CCAGCCCCGC CCCCGACCCG
2501 CCTGGCGGCC TCACCTACTT TCCCGTGGCC GACCTGTCTA TCATCGCCGC
2551 CCTCTATGCC CGCTTCACCG CCCAGATCCG AGGCGCCGTC GACCTGTCCC
2601 TCTATCCTCG AGAAGGGGGT GTCTCCAGCC GTGAGCTGGT GAAGAAGGTC
2651 TCCGATGTCA TATGGAACAG CCTCAGCCGC TCCTACTTCA AGGATCGGGC
2701 CCACATCCAG TCCCTCTTCA GCTTCATCAC AGGTTGGAGC CCAGTAGGTG
2751 GGAATCTTAT CCATGACCCA CTTCTTCAAA ACCCTCCATG GTTTACAGAA
2801 CCCTTTTAAG AACTGTAAGC CTTGTGAGGT TCGGCAGGTG TTATTTTCCT
2851 CTTTGCAGTT GGGAAACTGA AGCCCAGAGA GGGGAAATGA TATGCCAAAG
2901 TCACACACGG CATGGCAGGG CTGGAAGTGA AGCCTGATCA CTTGGCTCCA
2951 AATCATCAAC CTCACCTCTG CCCCCTCAGC ACCCCCACCC TTGCCACTGA
3001 ACAGCTACAG GAGTTCTAAG CATGAGACAC AGAGGGCGGC AGCAGATTTA
3051 GGGGGCAAGA AGATGAAATT GGGCTGCATT TGAGGCAGTT AAACAAAATA
3101 ATGGCTATGA AGATTTTTTT TTTTTTTTTT TTTTGAGACA GGGTCTCACT
3151 CTGTCCCCCA GGCTGGAGTG CAGTGGTGTG ATCATGGCTC ACTGCAGCCT
3201 CAGTCTCCCT GGGCTCAGAG ATCCTCCAAC CTCAGCCTCC TGAGTAGCTG
3251 AGAGTACAGG CATGCACCGT GGTGCTGGTT AATTTTTTGT ATTTTTTTTG

3301 TAGAGATGGT GTCTCACTAT GTGGCCCAGA CTGGTCTTGA ACTCTTGGGC
3351 TCAAGTGATC TGCCCGCCTC AGTCTCCCAA ATGCTGGGAT TACAGGTGTG
3401 AGCCACCGCA ACTGGTGGCC TATGAAAATT TTTTTTTTTT TTCAGACGGC
3451 GTCTCACTCT GTCGCCCAGG CTGGAGTGCA GTGGTGCAAT CTCGGCTCAC
3501 TGCAAGCTCT GCCTCCTGCT TTCATGCCAT TCTCCTGCCT CCTGCCTCAG
3551 CCTCCTGAGT AGCTGGGACT ACAGGAGCCT GCCACCATGC CTGGCTAATT
3601 TTTTTTTGGA TTTTTAGTAG AGACGAGGTT TCACCATGTT AGCCAGGATG
3651 GTCTCGATCT CCTGACCTCG TGATCCGCCC GCCTTGGCCT CCCAAAGTGC
3701 TGGGATTACA GGCGTGAGCC ACCGCACCTG GTCAAAAATG TTTGAGACAG
3751 AGAAGGGGCT TGACCTCAAA AGGCTTAAGA GTCAGGGCTT GCAAAGAGCT
3801 TTGCACCAAG CCCGGTTGAC TGGCAATCCC ATCCTGGTGT GCCATATTGA
3851 GAAGGAATCA GAGGCTGCTT CTCAGCTTAG CAGGAAAAGA GTGCAGAGAT
3901 AAATGAGGGT TATTTGTTGG TGGGTGTATA GCCAGAGAGT GTTGGCCAGC
3951 GTCCTGTTTT TGCCATTCCT GTTTTAACCT AGTAAGTGCA GTAAAATGGA
4001 ATCCCTAAAT CCATAGAATA TATAATAGAG TTGCAGAGAA AGACGAGGTA
4051 GGGCCAAAGG CTGGGTCAGC TACAGGATAT CCAGAAAGGT ATCTTGTTGG
4101 ACATAGAGGG TGTAAACAGG GAGAGAGTCT TTGAACACGT GGGAGGGAAG
4151 GGATGGAGGG ATAGTGGGCA GGAGAATCTG AGGTTGGGTC ACAGGCTTGG
4201 AAAGGGAGTG GGAGGGAGTG TGGCCCATCA CTACCTGGCC CCTTTCCCCA
4251 TGTTAAAGCA CAGAGGACCC TCTTTCATTA CCTCCCCCTT CCACAGGCAC
4301 CAAATTGGAC AGCTCCGGTG TGGCCTTTGC TGTGGTTGGG GCCTGCCAGG
4351 CCCTGGGTCT CCGGGATGTC CACCTCGCCC TGTCTGAGGA TCATGCCTGG
4401 GTAGTGTTTG GGCCCAATGG GGAGCAGACA GCTGAGGTCA CCTGGCACGG
4451 CAAGGGCAAC GAGGACCGCA GGGGCCAGAC AGTCAATGCC GGTGTGGCTG
4501 AGCGGGTATT GTTCCCTCCC CCCAGCCTTG TCCCCTTCAT ACTGTAGTAG
4551 CCCAAGCCAC CCAAGGGACT CCATTTTCTT GGGCCACACC CCTTTCTTCC
4601 CATCACCACC CACATAGGAA GGGAAGACAG AAGAGCCCCT TTTCCTGGCT
4651 GTCATTCCCT GAAGCAGGCA CAGGGTGGGC CATCATGAGA CATAATGATC
4701 TCATCCCCCC CTAAGAGCTG GCTGTACCTG AAAGGATCAT ACATGCGCTG
4751 TGACCGCAAG ATGGAGGTGG CGTTCATGGT GTGTGCCATC AACCCTTCCA
4801 TTGACCTGCA CACCGACTCG CTGGAGCTTC TGCAGCTGCA GCAGGTGAGG
4851 GCTGAGCCAA TGGGGCAGGA CTGGGCTAGG CCAGACTTGA CTTGCTGTGG
4901 GACCCTGGGC AGGGGCACTT TCCCTTCCTG AGCTTCAGCT TCCCCTCCTG
4951 GAAAAATGGG TTAGTAATTC CTGGCCTGGC CTTTCCCAGG GCTCTTGGGA
5001 GAGTAGAATT GAGATGTGAA ATTGCTTTGA CTCCATTAAA GGGCTGGTCC
5051 CAGAATTTTG GCCCTTCCAC ATGGTGGGTG GTCCCTGTTG GTTCTGACCC
5101 CCACCTCTGC CCGATAGGCT AAGGACCCGT TCTCCTCCCT GTTCCGTGGC
5151 TCATAACTCT CTCCTTCGGC TCCTAGAAGC TGCTCTGGCT GCTCTATGAC

5201 CTGGGACATC TGGAAAGGTC AGTAGAGGGA AGTGGCCAGG CTGCGCCTGG
5251 TGAGGCCGGG GGGCTGGGTG GCAGCCTGAA TTATGATCCT TTCCTAGGTA
5301 CCCCATGGCC TTAGGGAACC TGGCAGATCT AGAGGAGCTG GAGCCCACCC
5351 CTGGCCGGCC AGACCCACTC ACCCTCTACC ACAAGGTGGG GGCATCTAAG
5401 GAGGGTGCAG AAGGGAGACC CTAACAGTGG CTGAGGCAGG GGCCCTCATC
5451 TGGGCAGATG AGAAGAGAAC TTTGTGTGTT GGGGGGTATC GCCCATCCAG
5501 TCTCACTTTG TGTCAACTGT GTGCAGAATC AGTTCAGTCA GGGCTGTCTG
5551 AGGGGTGTCC AGGGTTCCCC AGCCTGGGAG TGGCAGGGGC TGCATTTGTC
5601 CCCTCAGCCC TGCCTTTTCT GCCACTGCTT ACTGTCCTTC CTGGAGTATA
5651 ACAGAGGTCA AATGTGGTAG GAGCACTGAA GAGGGGGTGT TCACTTGGTG
5701 GGTGTAGGTG GGGAGGAGGG CCATTGGGCT GGGCTTGAAA GTCTTTGGTG
5751 ATGTGTAGAA GAGTGTCTGA GAAAGAGAAG GGCCCTGAGC TCGGAGGGCA
5801 GGCCCCACCC CTGCAGTCTG CCCCAGGCCT CAGCCAGCAG TCCTGTAGAC
5851 CCAGGGAGGA GACCAGGTAG AAGGGCTGGC AGCGAGTGGA GGTGGGAGTG
5901 GAGATGGAGA GGACTCCCTG GGATCTTCCT GTGGCCCCTT CTGGGTGTGC
5951 CCTGGTGGGG CATTTGTGCC AGCAGGGCAG CTGGGGCTGC CTCCCTGAGG
6001 ATCCTCTGCC TCACCTCCAT CCAGGGCATT GCCTCAGCCA AGACCTACTA
6051 TCGGGATGAA CACATCTACC CCTACATGTA CCTGGCTGGC TACCACTGTC
6101 GCAACCGCAA TGTGCGGGAA GCCCTGCAGG CCTGGGCGGA CACGGCCACT
6151 GTCATCCAGG AGTGAGGATC CCCCTACTAG GGCCTGCAGC CTGTCCTTTC
6201 TTCCCCTCCA TCAGTTTCCA ACCACCCTCG TCCAGGACTG AGGCCTGGCT
6251 CCCACGCCCC ATCCCCTTTC CATCCAGTCC CTAGGCAGCA AGGCCACCAT
6301 TACCCAGGAG GTAGGGACCC TGATTAAGGT GTCACATCTT TCCCTCCCTC
6351 CCCTCTCCTC CTAATTTTTT TTTTCTCAGA ACAGTCTCAA ATCTCCAATG
6401 TTTAACCACC ATCATCCAGC AGTGGGACTT CCACCCTCGG CCCCATGCCC
6451 CCCTCCTCAT TCTTGCTTTC TTCCTCTGGG CTGACCCAGA CAGCATCATT
6501 TTGCAGTGAG GACCCCACCT ACTCCCCCAG CCCCTGGGGG CTCCATCCCC
6551 CGCCAGGTCC CTGGGGCTAC CCCCGATGGT GAGACCCCTT CAGACCCTAC
6601 AGAGACCCCA CTGCTCTCAC AGCTACAACT ACTGCCGGGA AGACGAGGAG
6651 ATCTACAAGG AGTTCTTTGA AGTAGCCAAT GATGTCATCC CCAACCTGCT
6701 GAAGGAGGCA GCCAGCTTGC TGGAGGCGGG CGAGGAGCGG CCGGGGGAGC
6751 AAAGCCAGGT GAAAGGCTGG AGCTCCAGCC TGTGTCCAGC CTCCCACCTG
6801 GACAGGGCTC CCTTCCACAG GGCCATGGGG GCTGCATGTA CGGGATTAGG
6851 GATGGCAGGA GGAAGGTGGC CCTGAGCAGA CAGCTATGTT CCCTTTTGCT
6901 ATAACTGAGG TCCTGGGCCC ACGTTGGACG GGACTGAAGG TATTTTAGAG
6951 GTTTCTACCC TGTGCCTTCA GTTTCATGGC CAGACTCCCT CCCTCAGCTG
7001 AGGGGTGGAG GTAGGGATGG TACGTCCTGG CTATGGATTG GCTTTATAAA
7051 AGGAAAGAGG TTCTAAGAAT GTTCCCAACC TATGCTTACC TTTTCTGGAG

7101 CCAGGGGTCT TTGCCTAGGT GGGGGGCCTG GCCTGTGCCC TCTGCTAAGG
7151 GGTGAGTAAG AGACTGATCT GTGCCCTCCC TTCCCCCTCG TCCAGGGCAC
7201 CCAGAGCCAA GGTTCCGCCC TCCAGGACCC TGAGTGCTTC GCCCACCTGC
7251 TGCGATTCTA CGACGGCATC TGCAAATGGG AGGAGGGCAG TCCCACGCCT
7301 GTGCTGCACG TGGGCTGGGC CACCTTTCTT GTGCAGTCCC TAGGCCGTTT
7351 TGAGGGACAG GTGAGGGACA GCTGCACAGA GGTCTGGGCA CTACAGGTGG
7401 TGACAGCAGC CACGGGCTTG TCAGACTTTT CTGGCCCAGG GGCAGCATCT
7451 GCCCATCCCC TTCGGTGCCG ATGGGACTGA GACCCCCTGG GTGGGATGGG
7501 ATGGCCAGAG CAGGGTCCTG GAGTTCCAGC CACTGGCCGG CAACCTTGCT
7551 CTCACCTTGC TCTCCCCACT GGCCCAGGTG CGGCAGAAGG TGCGCATAGT
7601 GAGCCGAGAG GCCGAGGCGG CCGAGGCCGA GGAGCCGTGG GGCGAGGAAG
7651 CCCGGGAAGG CCGGCGGCGG GGCCCACGGC GGGAGTCCAA GCCAGAGGAG
7701 CCCCCGCCGC CCAAGAAGCC AGCACTGGAC AAGGGCCTGG GCACCGGCCA
7751 GGGTGCAGTG TCAGGACCCC CCCGGAAGCC TCCTGGGACT GTCGCTGGCA
7801 CAGCCCGAGG CCCTGAAGGT GGCAGCACGG CTCAGGTGCC AGCACCCGCA
7851 GCATCACCAC CGCCGGAGGG TCCAGTGCTC ACTTTCCAGA GTGAGAAGAT
7901 GAAGGGCATG AAGGAGCTGC TGGTGGCCAC CAAGATCAAC TCGAGCGCCA
7951 TCAAGCTGCA ACTCACGGCA CAGTCGCAAG TGCAGATGAA GAAGCAGAAA
8001 GTGTCCACCC CTAGTGACTA CACTCTGTCT TTCCTCAAGC GGCAGCGCAA
8051 AGGCCTCTGA ACTACTGGGG ACTTCGGACC GCTTGTGGGG ACCCAGGCTC
8101 CGCCTTAGTC CCCCAACTCT GAGCCCATGT TCTGCCCCCA GCCCAAAGGG
8151 GACAGGCCTC ACCTCTACCC AAACCCTAGG TTCCCGGTCC CGAGTACAGT
8201 CTGTATCAAA CCCACGATTT TCTCCAGCTC AGAACCCAGG GCTCTGCCCC
8251 AGTCGTTAGA ATATAGGTCT CTTCTCCCAG AATCCCAGCC GGCCAATGGA
8301 AACCTCACGC TGGGTCCTAA TTACCAGTCT TTAAAGGCCC AGCCCCTAGA
8351 AACCCAAGCT CCTCCTCGGA ACCGCTCACC TAGAGCCAGA CCAACGTTAC
8401 TCAGGGCTCC TCCCAGCTTG TAGGAGCTGA GGTTTCACCC TTAACCCAAG
8451 GGAGCACAGG TCCCACCTCC AGCCCGGGGA GCCTAGGACC ACTCAGCCCC
8501 TAGGAGTATA TTTCCGCACT TCAGAATTCC ATATCTTGCG AATCCAAGCT
8551 CCCTGCCCCA AATAACTTCA GTCCTGCTTC CAGAATTTGG AAATCCTAGT
8601 TTCCTCTCCT TCGTATCCCG AGTCTGGGAC ACAAAACTCC GCCCCCAGCC
8651 TATGAGCATC CTGAGCCCCG CCCTCTTCCT GACGAAACTG GCCCCGGATC
8701 AGAGCAGGAC CTCCCTTCCG ACCCTCTGGG AACCTCCCAG AGGTCCAGCC
8751 CATCTCGGAG CATCCCGGAG GAAATCTGCA GAGGGGTTAG GAGTGGGTGA
8801 CAAGAGCCTG ATCTCTTCCT GTTTTGTACA TAGATTTATT TTTCAGTTCC
8851 AAGAAAGATG AATACATTTT GTTAAAAAAA ATATAAAGCG CAAGTCCATG
8901 TTTATCTGGG AAATTGGGGA TGGGGCGGGG AGTGGAGCGC CCCTTCTTCC
8951 CTTTGTCTTC TGGCTCCCGG GACTTTGCGC TCCCTACCTG TGGAGCGCGA

9001 GCGACAGTGG CGGCGGAAGG ACGTAGGCTC CGCCCCGGCC TTGGGGCTTC
9051 CCCCGCGCCG CCGAGGGCCC GTCCCGCGGG CGCCTCCTCC CGGACTGGCG
9101 GTGGGGCATC CCNGGGCGCG GCCCCGCCCC CGGGCTTCAG CCCCGCCCCC
9151 GCGGCTTCAG AGCCACGGGC GCCCGCCCCG

## Claims

1. An isolated or recombinant protein defined as follows:
i) having a calculated molecular weight of about 67.5 kDa; and
ii) having at least 60% amino acid sequence identity to a protein with a sequence as set forth in SEQ ID NO: 2.

2. The isolated or recombinant protein of claim 1, wherein the protein has at least 80% amino acid sequence identity to a protein with a sequence as set forth in SEQ ID NO: 2.

3. The isolated or recombinant protein of claim 2, wherein the protein has a sequence as set forth in SEQ ID NO: 2.

4. An isolated or recombinant nucleic acid associated with the presence of multiple endocrine neoplasia type 1, wherein said nucleic acid encodes a protein as defined in any one of claims 1 to 3.

5. The isolated or recombinant nucleic acid of claim 4, which further comprises non-coding sequence.

6. The isolated or recombinant nucleic acid of claim 5, wherein the non-coding sequence comprises introns.

7. The isolated or recombinant nucleic acid of any one of claims 4 to 6, wherein said nucleic acid comprises the sequence of SEQ ID NO: 3.

8. The isolated or recombinant nucleic acid of either one of claims 4 or 5, wherein said nucleic acid comprises the sequence of SEQ ID NO: 1.

9. The isolated or recombinant nucleic acid of any one of claims 4 to 6, wherein the nucleic acid sequence specifically hybridizes to SEQ ID NO: 1 under stringent conditions, wherein the hybridization conditions comprise 50% formamide at 42°C and the wash conditions comprise 0.2 x SSC at 65°C for 15 minutes.

10. An antibody, specifically immunoreactive under immunologically reactive conditions, to a protein which has an amino acid sequence as set forth in SEQ ID NO. 2.

11. A method for detecting the presence of menin in a human cell or tissue in a biological sample isolated from a human, wherein menin is a protein encoded by a DNA sequence as set forth in SEQ ID NO. 1 (*MEN1* cDNA) or SEQ ID NO. 3, or by a nucleic acid selectively hybridising to the exons of SEQ ID NO. 3 under hybridisation conditions comprising 50% formamide at 42°C and wash conditions comprising 0.2 X SSC at 65°C for 15 minutes, said method comprising:
i) contacting the biological sample with a menin specific reagent; and
ii) detecting the level of menin specific reagent that selectively associates with the sample.

12. The method of claim 11, wherein the menin specific reagent is selected from the group comprising: menin specific antibodies, *MEN1* amplification primers and nucleic acid probes which selectively bind to *MEN1*.

13. The method of claims 11 or 12, wherein the contacting step uses a menin specific antibody.

14. The method of any one of claims 11 to 13, wherein the human from which the sample was isolated is suspected of being at risk from multiple endocrine neoplasia type 1.

15. The method of any one of claims 11 to 14, wherein the contacting step uses a *MEN1* specific PCR primer pair that amplifies a region of the *MEN1* gene in which a mutation has been associated with multiple endocrine neoplasia type 1.

16. A method for detecting in a test sample the presence or absence of a mutation in a nucleotide sequence essentially encoding human menin or the presence or absence of *MEN1* allele, wherein menin is a protein encoded by a DNA sequence as set forth in SEQ ID NO. 1 (*MEN1* cDNA) or SEQ ID NO. 3, or by a nucleic acid selectively hybridising to the exons of SEQ ID NO. 3 under hybridisation conditions comprising 50% formamide at 42°C and wash conditions comprising 0.2 X SSC at 65°C for 15 minutes, said method comprising:
(a) contacting said test sample suspected of containing a gene missing a MEN1 allele or encoding a mutant form of the human menin with a first oligonucleotide having a sequence competent to discriminate between the wild type gene and the missing allele or mutant form, and
(b) detecting the formation of a duplex between the gene and the first oligonucleotide sequence.

17. A method of claim 16, wherein the first oligonucleotide is unable to bind to the wild-type *MEN1* gene under hybridization conditions in which the first oligonucleotide binds to the mutant sequence of *MEN1*.

18. A method of either one of claims 16 or 17, wherein the contacting step further comprises amplifying a portion of the human *MEN1* gene and where the first oligonucleotide is a polymerase chain reaction amplification primer which binds to an intron of *MEN1*.

19. A method of either of claims 16 or 17, wherein the contacting step further comprises amplifying a portion of MEN1 and where the first oligonucleotide is a polymerase chain reaction amplification primer which discriminates between wild-type and mutant forms of *MEN1* using allelic specific polymerase chain reaction.

20. A method of claim 19, wherein the first oligonucleotide binds to either exons or introns of the genomic DNA encoding the human menin gene.

21. A kit for detecting in a test sample the presence or absence of a mutation in a nucleotide sequence essentially encoding menin, wherein menin is a protein encoded by a DNA sequence as set forth in SEQ ID NO. 1 (*MEN1* cDNA) or SEQ ID NO. 3, or by a nucleic acid selectively hybridising to the exons of SEQ ID NO. 3 under hybridisation conditions comprising 50% formamide at 42°C and wash conditions comprising 0.2 X SSC at 65°C for 15 minutes, said kit comprising:
(a) a container holding a first nucleotide sequence whereby said first nucleotide sequence is capable of discriminating between the wild type gene and the mutant form; and
(b) a container holding a reagent for detecting the formation of a duplex between the gene and the first nucleotide sequence.

22. A kit as claimed in claim 21 wherein said nucleotide sequence under test corresponds to the wild type allele encoding menin.

23. The kit of either of claim 21 or 22, further comprising amplification primer pairs specifically binding to a human genomic DNA sequence containing *MEN1*.

24. A kit for detecting the presence or absence of menin in persons at risk for multiple endocrine neoplasia type 1, wherein menin is a protein encoded by a DNA sequence as set forth in SEQ ID NO. 1 (*MEN1* cDNA) or SEQ ID NO. 3, or by a nucleic acid selectively hybridising to the exons of SEQ ID NO. 3 under hybridisation conditions comprising 50% formamide at 42°C and the wash conditions comprising 0.2 X SSC at 65°C for 15 minutes, said kit comprising a first container containing an antibody specific for menin or an antibody as claimed in claim 10 and a second container containing an antigen that specifically binds to the said antibody in the first container.

25. The kit of claim 24, wherein the antibody can detect the presence of a wild-type menin protein.

26. The kit of claim 24, wherein the antibody can detect the presence of a mutated menin protein.

27. A transfected cell comprising a heterologous nucleic acid as defined in any one of claims 4 to 9.

28. A transfected cell into which an exogenous nucleic acid sequence has been introduced, wherein the exogenous nucleic acid specifically hybridizes under stringent conditions to SEQ ID NO. 1 or SEQ ID NO. 3 and wherein the hybridisation conditions comprise 50% formamide at 42°C and the wash conditions comprise 0.2 X SSC at 65°C for 15 minutes.

29. The transfected cell of claim 27 or claim 28, wherein the cell is a human cell.

30. A non-human organism into which an exogenous nucleic acid sequence has been introduced, wherein the exogenous nucleic acid comprises a nucleic acid as defined in any one of claims 4 to 9, wherein the organism expresses the exogenous nucleic acid as a menin protein.

31. An expression cassette comprising a nucleic acid as defined in any one of claims 4 to 9, wherein the nucleic acid is operably linked to a promoter.

32. The expression cassette of claim 31, further comprising an expression vector.

## Patentansprüche

1. Isoliertes oder rekombinantes Protein, das wie folgt definiert ist:
i) es weist ein berechnetes Molekulargewicht von etwa 67,5 kDa auf;
ii) es hat eine wenigstens 60 %ige Aminosäuresequenzidentität mit einem Protein mit einer Sequenz gemäß SEQ ID NO:2.

2. Isoliertes oder rekombinantes Protein nach Anspruch 1, wobei das Protein eine wenigstens 80%ige Aminosäuresequenzidentität mit einem Protein mit einer Sequenz gemäß SEQ ID NO:2 aufweist.

3. Isoliertes oder rekombinantes Protein nach Anspruch 2, wobei das Protein eine Sequenz gemäß SEQ ID NO:2 aufweist.

4. Isolierte oder rekombinante Nukleinsäure, welche mit dem Vorliegen von multipler endokriner Neoplasie Typ I assoziiert ist, wobei die Nukleinsäure für ein Protein gemäß der Definition in einem der Ansprüche 1 bis 3 kodiert.

5. Isolierte oder rekombinante Nukleinsäure nach Anspruch 4, welche außerdem eine nicht-kodierende Sequenz umfasst.

6. Isolierte oder rekombinante Nukleinsäure nach Anspruch 5, wobei die nicht-kodierende Sequenz Introns umfasst.

7. Isolierte oder rekombinante Nukleinsäure nach einem der Ansprüche 4 bis 6, wobei die Nukleinsäure die Sequenz gemäß SEQ ID NO:3 umfasst.

8. Isolierte oder rekombinante Nukleinsäure nach einem der Ansprüche 4 oder 5, wobei die Nukleinsäure die Sequenz gemäß SEQ ID NO:1 umfasst.

9. Isolierte oder rekombinante Nukleinsäure nach einem der Ansprüche 4 bis 6, wobei die Nukleinsäuresequenz spezifisch mit SEQ ID NO:1 unter stringenten Bedingungen hybridisiert, wobei die Hybridisierungsbedingungen 50% Formamid bei 42°C und die Waschbedingungen 0,2 x SSC bei 65°C für 15 Minuten umfassen.

10. Antikörper, der unter immunologisch reaktiven Bedingungen mit einem Protein spezifisch immunreaktiv ist, welches eine Aminosäuresequenz gemäß SEQ ID NO:2 aufweist.

11. Verfahren zur Detektion der Präsenz von Menin in einer menschlichen Zelle oder einem menschlichen Gewebe in einer biologischen Probe, welche aus dem Menschen isoliert worden ist, wobei Menin ein Protein ist, das durch eine DNA Sequenz gemäß SEQ ID NO:1 (MEN1 cDNA) oder SEQ ID NO:3 kodiert wird oder von einer Nukleinsäure kodiert wird, welche mit den Exons von SEQ ID NO:3 unter Hybridisierungsbedingungen, umfassend 50% Formamid bei 42°C und Waschbedingungen, umfassend 0,2 x SSC bei 65°C für 15 Minuten, selektiv hybridisiert, wobei man bei diesem Verfahren:
i) die biologische Probe mit einem Menin-spezifischen Reagenz in Kontakt bringt; und
ii) den Gehalt von Menin-spezifischem Reagenz, das mit der Probe selektiv assoziiert, detektiert.

12. Verfahren nach Anspruch 11, wobei das Menin-spezifische Reagenz ausgewählt ist unter: Menin-spezifischen Antikörpern, MEN1-Amplifikationsprimern und Nukleinsäuresonden, welche selektiv an MEN1 binden.

13. Verfahren nach Anspruch 11 oder 12, wobei für den Schritt des in Kontakt bringens ein Menin-spezifischer Antikörper verwendet wird.

14. Verfahren nach einem der Ansprüche 11 bis 13, wobei der Mensch, aus dem die Probe isoliert worden ist, vermutlich ein Risiko für multiple endokrine Neoplasie Typ I ausweist.

15. Verfahren nach einem der Ansprüche 1 bis 14, wobei der Schritt des in Kontakt bringens ein MEN1-spezifisches PCR-Primerpaar umfasst, welches eine Region des MEN1-Gens amplifiziert, in welcher eine Mutation multipler endokriner Neoplasie Typ I zugeordnet wurde.

16. Verfahren zum Nachweis des Vorliegens oder Fehlens einer Mutation in einer Nukleotidsequenz, welche im Wesentlichen menschliches Menin kodiert oder des Vorliegens oder Fehlens eines MEN1-Allels in einer Testprobe, wobei Menin ein Protein ist, welches von einer DNA-Sequenz gemäß SEQ ID NO:1 (MEN1 cDNA) oder SEQ ID NO:3 oder einer Nukleinsäure kodiert wird, welche selektiv mit den Exons gemäß SEQ ID NO:3 unter Hybridisierungsbedingungen, umfassend 50% Formamid bei 42°C und Waschbedingungen, umfassend 0,2 x SSC bei 65°C für 15 Minuten, selektiv hybridisiert, wobei man bei diesem Verfahren:
(a) die Testprobe, von der man vermutet, dass sie ein Gen enthält, dem ein MEN1-Allel fehlt oder das für eine mutante Form von humanem Menin kodiert, mit einem ersten Oligonukleotid, das eine Sequenz aufweist, die zur Diskriminierung zwischen dem Wildtypgen und dem fehlenden Allel oder der mutanten Form befähigt ist, in Kontakt bringt, und
(b) die Bildung eines Duplex zwischen dem Gen und der ersten Oligonukleotidsequenz nachweist.

17. Verfahren nach Anspruch 16, wobei das erste Oligonukleotid nicht zur Bindung an das Wildtyp MEN1-Gen unter Hybridisierungsbedingungen befähigt ist, bei denen das erste Oligonukleotid an die Mutantensequenz von MEN1 bindet.

18. Verfahren nach einem der Ansprüche 16 und 17, wobei der Schritt des in Kontakt bringens außerdem die Amplifizierung eines Teils des humanen MEN1-Gens umfasst und wobei das erste Oligonukleotid ein Polymerasekettenreaktion-Amplifikationsprimer ist, der an ein Intron von MEN1 bindet.

19. Verfahren nach einem der Ansprüche 16 und 17, wobei der Schritt des in Kontakt bringens außerdem die Amplifizierung eines Teils von MEN1 umfasst und wobei das erste Oligonukleotid ein Polymerasekettenreaktion-Amplifikationsprimer ist, der zwischen Wildtyp und mutanten Formen von MEN1 unter Verwendung einer Allel-spezifischen Polymerasekettenreaktion diskriminiert.

20. Verfahren nach Anspruch 19, wobei das erste Oligonukleotid entweder an Exons oder Introns der genomischen DNA bindet, welche das menschliche Menin-Gen kodiert.

21. Kit zum Nachweis des Vorliegens oder Fehlens einer Mutation in einer Nukleotidsequenz, welche im Wesentlichen für Menin kodiert in einer Testprobe, wobei Menin ein Protein ist, das von einer DNA Sequenz gemäß SEQ ID NO:1 (MEN1 cDNA) oder SEQ ID NO:3 oder von einer Nukleinsäure kodiert wird, welche mit Exons von SEQ ID NO:3 unter Hybridisierungsbedingungen, umfassend 50% Formamid bei 42°C und Waschbedingungen, umfassend 0,2 x SSC bei 65°C für 15 Minuten, selektiv hybridisiert, wobei der Kit umfasst:
(a) einen Behälter, enthaltend eine erste Nukleotidsequenz, wobei diese erste Nukleotidsequenz befähigt ist zur Diskriminierung zwischen dem Wildtypgen und der mutanten Form;
(b) einen Behälter, enthaltend ein Reagenz für den Nachweis der Bildung eines Duplex zwischen dem Gen und der ersten Nukleotidsequenz.

22. Kit nach Anspruch 21, wobei die zu testende Nukleotidsequenz dem Wildtyp-Allel entspricht, das für Menin kodiert.

23. Kit nach Anspruch 21 oder 22, weiterhin umfassend Amplifikations-Primerpaare, welche spezifisch an eine menschliche genomische DNA Sequenz, enthaltend MEN1 binden.

24. Kit zum Nachweis des Vorliegens oder Fehlens von Menin in Personen mit einem Risiko für multiple endokrine Neoplasie Typ I, wobei Menin ein Protein ist, das von einer DNA Sequenz gemäß SEQ ID NO:1 (MEN1 cDNA) oder SEQ ID NO:3 oder von einer Nukleinsäure kodiert wird, welche mit Exons von SEQ ID NO:3 unter Hybridisierungsbedingungen, umfassend 50% Formamid bei 42°C und Waschbedingungen, umfassend 0,2 x SSC bei 65°C für 15 Minuten, selektiv hybridisiert, wobei der Kit einen ersten Behälter umfasst, der einen Antikörper mit Spezifität für Menin oder einen Antikörper nach Anspruch 10 enthält, und einen zweiten Behälter umfasst, der ein Antigen enthält, der spezifisch an den Antikörper im ersten Behälter bindet.

25. Kit nach Anspruch 24, wobei der Antikörper das Vorliegen eines Wildtyp-Meninproteins detektiert kann.

26. Kit nach Anspruch 24, wobei der Antikörper das Vorliegen eines mutierten Meninproteins detektieren kann.

27. Transfizierte Zelle, umfassend eine heterologe Nukleinsäure gemäß der Definition in einem der Ansprüche 4 bis 9.

28. Transfizierte Zelle, in welche eine exogene Nukleinsäuresequenz eingeführt wurde, wobei die exogene Nukleinsäure unter stringenten Bedingungen spezifisch mit SEQ ID NO:1 oder SEQ ID NO:3 hybridisiert, und wobei die Hybridisierungsbedingungen 50% Formamid bei 42°C umfassen und die Waschbedingungen 0,2 x SSC bei 65°C für 15 Minuten umfassen.

29. Transfizierte Zelle nach Anspruch 27 oder Anspruch 28, wobei die Zelle eine humane Zelle ist.

30. Nicht-menschlicher Organismus, in welchen eine exogene Nukleinsäuresequenz eingeführt wurde, wobei die exogene Nukleinsäuresequenz eine Nukleinsäure gemäß der Definition in einem der Ansprüche 4 bis 9 umfasst, wobei der Organismus die exogene Nukleinsäure als Meninprotein exprimiert.

31. Expressionskassette umfassend eine Nukleinsäuresequenz gemäß der Definition in einem der Ansprüche 4 bis 9, wobei die Nukleinsäure mit einem Promotor operativ verknüpft ist.

32. Expressionskassette nach Anspruch 31, außerdem umfassend einen Expressionsvektor.

## Revendications

1. Une protéine isolée ou recombinante définie comme suit:
i) ayant une masse moléculaire calculée d'environ 67,5 kDa; et
ii) ayant une identité de séquence des acides aminés d'au moins 60% avec une protéine ayant la séquence ainsi que montrée dans SEQ ID No: 2.

2. La protéine isolée ou recombinante de la revendication 1, dans laquelle la protéine a une identité de séquence des acides aminés d'au moins 80% avec une protéine ayant la séquence ainsi que montrée dans SEQ ID No: 2.

3. La protéine isolée ou recombinante de la revendication 2, dans laquelle la protéine a la séquence montrée dans SEQ ID No: 2.

4. Un acide nucléique isolé ou recombinant associé à la présence d'une adénomatose polyendocrinienne de type 1, dans lequel l'acide nucléique code pour une protéine ainsi que définie dans l'une quelconque des revendications 1 à 3.

5. L'acide nucléique isolé ou recombinant de la revendication 4, qui comprend de plus une séquence non codante.

6. L'acide nucléique isolé ou recombinant de la revendication 5, dans lequel la séquence non codante comporte des introns.

7. L'acide nucléique isolé ou recombinant selon l'une quelconque des revendications 4 à 6, dans lequel cet acide nucléique comprend la séquence SEQ ID No: 3.

8. L'acide nucléique isolé ou recombinant selon l'une quelconque des revendications 4 ou 5, dans lequel cet acide nucléique comprend la séquence SEQ ID No: 1.

9. L'acide nucléique isolé ou recombinant selon l'une quelconque des revendications 4 à 6, dans lequel la séquence de l'acide nucléique s'hybride spécifiquement à SEQ ID No: 1 en conditions stringentes, dans lesquelles les conditions d'hybridation comprenant du formamide à 50 % à 42°C et les conditions de lavage consistent en 0,2 x SSC à 65°C pendant 15 minutes.

10. Un anticorps, spécifiquement immunoréactif en conditions de réactivité immunologiques, à l'encontre d'une protéine qui a une séquence d'acides aminés ainsi que montrée dans SEQ ID No. 2.

11. Un procédé pour détecter la présence de ménine dans une cellule ou un tissu humain dans un échantillon biologique isolé à partir d'un humain, dans lequel la ménine est une protéine codée par une séquence d'ADN ainsi que montrée dans SEQ ID No. 1 (ADNc *MEN1*) ou SEQ ID No. 3, ou bien par un acide nucléique s'hybridant sélectivement aux exons de SEQ ID No. 3 en des conditions d'hybridation comprenant du formamide à 50% à 42°C et des conditions de lavage consistant en 0,2 x SSC à 65°C pendant 15 minutes, ce procédé comprenant le fait :
i) de mettre l'échantillon biologique en contact avec un réactif spécifique de la ménine; et
ii) de détecter la quantité de réactif spécifique à la ménine qui s'associe sélectivement à l'échantillon.

12. Le procédé selon la revendication 11, dans lequel le réactif spécifique de la ménine est choisi dans le groupe consistant en: les anticorps spécifiques contre la ménine, les amorces d'amplification *MEN1* et les sondes d'acide nucléique qui se fixent sélectivement à *MEN1*.

13. Le procédé selon les revendications 11 ou 12, dans lequel l'étape de mise en contact utilise un anticorps spécifique contre la ménine.

14. Le procédé selon l'une quelconque des revendications 11 à 13, dans lequel on soupçonne l'humain à partir duquel l'échantillon a été isolé d'être à risque pour une adénomatose polyendocrinienne de type 1.

15. Le procédé de l'une quelconque des revendications de 11 à 14, dans lequel l'étape de mise en contact utilise une paire d'amorces pour la réaction d'amplification en chaîne par polymérase spécifique pour *MEN1* qui amplifie une région du gène MEN 1 dans lesquelles une mutation a été associée avec l'adénomatose polyendocrinienne de type 1.

16. Un procédé pour détecter dans un échantillon devant être testé la présence ou l'absence d'une mutation dans une séquence de nucléotides qui code essentiellement pour la ménine humaine ou bien la présence ou l'absence d'un allèle *MEN1*, dans lequel la ménine est une protéine qui est codée par une séquence d'ADN telle que montrée dans SEQ ID No. 1 (ADNc *MEN1*) ou SEQ ID No. 3, ou bien par un acide nucléique s'hybridant sélectivement aux exons de SEQ ID No. 3 en des conditions d'hybridation comprenant du formamide à 50 % à 42°C et des conditions de lavage consistant en 0,2 x SSC à 65°C pendant 15 minutes, ce procédé comprenant le fait :
(a) de mettre l'échantillon, qui doit être testé et dont on suspecte qu'il contient un gène manquant d'un allèle MEN1 ou qui code pour une forme mutante de la ménine humaine, en contact avec un premier oligonucléotide ayant une séquence capable de discriminer entre le gène sauvage et l'allèle manquant ou la forme mutante, et
(b) de détecter la formation d'un duplex entre le gène et la première séquence d'oligonucléotides.

17. Un procédé selon la revendication 16, dans lequel le premier oligonucléotide est incapable de se fixer au gène *MEN1* sauvage en des conditions d'hybridation dans lesquelles le premier oligonucléotide se fixe à la séquence mutante de *MEN1*.

18. Un procédé selon l'une quelconque des revendications 16 ou 17, dans lequel l'étape de mise en contact comprend de plus l'amplification d'une portion du gène *MEN1* humain et dans lequel le premier oligonucléotide est une amorce pour la réaction d'amplification en chaîne par polymérase qui se fixe à un intron de *MEN1*.

19. Un procédé selon l'une quelconque des revendications 16 ou 17, dans lequel l'étape de mise en contact consiste de plus en l'amplification d'une portion de MEN1 et dans lequel le premier oligonucléotide est une amorce pour la réaction d'amplification en chaîne par polymérase qui est capable de discrimination entre les formes sauvages et mutantes de *MEN1* en utilisant une réaction d'amplification en chaîne par polymérase spécifique à l'un des allèles.

20. Un procédé selon la revendication 19, dans lequel le premier oligonucléotide se fixe aux introns ou aux exons de l'ADN génomique qui code pour le gène de la ménine humaine.

21. Une trousse pour détecter dans un échantillon devant être testé la présence ou l'absence d'une mutation dans une séquence de nucléotides qui code essentiellement pour la ménine, dans laquelle la ménine est une protéine codée par une séquence d'ADN ainsi que montrée dans SEQ ID No. 1 (ADNc *MEN1*) ou SEQ ID No 3, ou bien par un acide nucléique qui s'hybride sélectivement aux exons de SEQ ID No. 3 en des conditions d'hybridation comprenant du formamide à 50% à 42°C et des conditions de lavage consistant en 0,2 x SSC à 65°C pendant 15 minutes, cette trousse comprenant :
(a) un récipient contenant une première séquence de nucléotides dans laquelle cette première séquence de nucléotides est capable de discriminer entre le gène sauvage et la forme mutante; et
(b) un récipient contenant un réactif pour détecter la formation d'un duplex entre le gène et la première séquence de nucléotides.

22. Une trousse ainsi que revendiquée dans la revendication 21 dans laquelle la séquence de nucléotides testée correspond à l'allèle sauvage codant pour la ménine.

23. Une trousse selon l'une quelconque des revendications 21 ou 22, qui comprend de plus des paires d'amorces pour amplification qui se fixent spécifiquement à une séquence d'ADN génomique humain contenant *MEN1*.

24. Une trousse pour détecter la présence ou l'absence de ménine chez des personnes à risque pour une adénomatose polyendocrinienne de type 1, dans laquelle la ménine est une protéine codée par une séquence d'ADN ainsi que montrée dans SEQ ID No. 1 (ADNc *MEN1*) ou SEQ ID No. 3, ou bien par un acide nucléique qui s'hybride sélectivement aux exons de SEQ ID No. 3 en des conditions d'hybridation consistant en 50% de formamide à 42°C et des conditions de lavage consistant en 0,2 x SSC à 65°C pendant 15 minutes, cette trousse comprenant un premier récipient contenant un anticorps contre la ménine spécifique ou un anticorps ainsi que revendiqué dans la revendication 10 et un second récipient contenant un antigène qui se fixe spécifiquement à l'anticorps du premier récipient.

25. Une trousse selon la revendication 24, dans laquelle l'anticorps peut détecter la présence d'une protéine de ménine sauvage.

26. La trousse selon la revendication 24, dans laquelle l'anticorps peut détecter la présence d'une protéine de ménine mutée.

27. Une cellule transfectée consistant en un acide nucléique hétérologue ainsi que défini dans l'une quelconque des revendications de 4 à 9.

28. Une cellule transfectée dans laquelle une séquence d'acide nucléique exogène a été introduite, dans laquelle l'acide nucléique exogène s'hybride spécifiquement en conditions stringentes à SEQ ID No. 1 ou à SEQ ID No. 3 et dans laquelle les conditions d'hybridation comprenant du formamide à 50% à 42°C et les conditions de lavage consistent en 0,2 x SSC à 65°C pendant 15 minutes.

29. La cellule transfectée de la revendication 27 ou de la revendication 28, dans laquelle la cellule est une cellule humaine.

30. Un organisme non humain dans lequel une séquence d'acide nucléique exogène a été introduite, dans lequel l'acide nucléique exogène consiste en un acide nucléique ainsi que défini dans l'une quelconque des revendications 4 à 9, dans lequel l'organisme exprime l'acide nucléique exogène sous forme d'une protéine de ménine.

31. Une casette d'expression consistant en un acide nucléique ainsi que défini par l'une quelconque des revendications 4 à 9, dans laquelle l'acide nucléique est lié de manière fonctionnelle à un promoteur.

32. La cassette d'expression de la revendication 31, qui comprend de plus un vecteur d'expression.
